# EUROPEAN PATENT SPECIFICATION

(11) **EP 1 161 191 B1**
(45) Date of publication and mention of the grant of the patent: **20.02.2008**
(21) Application number: 00909555.5
(22) Date of filing: 15.03.2000
(51) Int. Cl.: A61B 17/70

(54) **SPINAL OSTEOSYNTHESIS INSTRUMENTATION**
INSTRUMENTE ZUR OSTEOSYNTHESE DER WIRBELSÄULE
INSTRUMENTS D'OSTEOSYNTHESE VERTEBRALE

(30) Priority: 16.03.1999 FR 9903234
(43) Date of publication of application: 12.12.2001
(73) Proprietor: SOCIETE DE FABRICATION DE MATERIEL ORTHOPEDIQUE SOFAMOR, 93290 Tremblay-en-France (FR)
(72) Inventor: Barbera-Alacreu, Jose Vicente, Hosp. Gen. Univ., 46014 Valencia (ES); Graziani, Noel, Clinique de la Residence du Parc, 13362 Marseille Cedex 10 (FR); Gournay, Jose, F-77230 Dammartin en Goele (FR); Malandain, Hugues, F-13300 Salon de Provence (FR)
(74) Representative: Neyret, Daniel Jean Marie
(86) International application number: PCT/IB2000/000281
(87) International publication number: WO 2000/054681

(56) References cited:
- EP-A- 0 507 162
- EP-A- 0 628 289
- EP-A- 0 739 610
- WO-A-95/35067
- WO-A-98/41160
- FR-A- 2 697 993
- FR-A- 2 758 971
- FR-A- 2 767 669
- US-A- 5 129 899

## Description

The present application claims the priority of French Patent Application No. 99 03 234, filed 16 March 1999.

### BACKGROUND OF THE INVENTION

The present invention relates to an instrument for the osteosynthesis of a spinal segment, and more particularly to instruments for the connection of vertebral deformations and degradations. Among portions of the spine to which these devices may be particularly adopted include the thoracic, thoraco-lumbar, and lumbar spinal segments.

Systems are known in the art that employ elongated plates or rods to interconnect a series of bone anchors, such as, for example, pedicular screws, to correct spinal deformities or degradation.

European Patent No. 0,383,992 and U.S. Patent No. 4,611,581 each describe instruments in which spinal plates are bent to conform with the anatomy of a particular spinal segment, and include pedicular screws having heads extended at the end opposite their bone anchor portion by a threaded end adapted to receive a nut for rigidly securing the plate to the screw. An elongate opening in the spinal plates enables adjustability of the position of the screw relative to the plate.

Until now, most instruments of the above type have included 90º angular connections between the screw and the plates, with the plates typically being bent to conform to the local lordosis or cyphosis. The vertebral plates generally have a length spanning two or three vertebral levels. Larger spans are typically avoided due to difficulties encountered in bending the plates. Additionally, titanium alloy plates or titanium plates tend to weaken if they are bent at a sharp angle when cold. This weakening causes the appearance of splits and/or cracks, and commensurately reduces the fatigue resistance of the plates.

Thus, prior plate-type spinal osteosynthesis instrumentation is not well suited for spanning across several levels of vertebral bodies. Also, prior instrumentation does not adequately accommodate for variations in the alignment of the screws in the frontal plane. Additionally, existing instrumentation is not capable of adequately conforming to the anatomy of the local lordosis or cyphosis. This difficulty can only be overcome by bending the plate into the desired configuration. Finally, existing instrumentation does not adequately accommodate for any angular variation in the convergence of the pedicular screws.

Document WO-A-95/35067 discloses an implant device which has all the features of the preamble of claim 1.

Document EP-A-0739610 describes an apparatus for maintaining vertebrae in a desired spatial relationship. Document WO-A-98/41160 describes a cervical fixation system. These documents show connector members which can slide on a first member, such as a plate. But these connector members are not captured on a face of the first member in a direction substantially perpendicular to a longitudinal axis of the first member.

One object of the present invention is to provide spinal instrumentation that allows relative articulation between a screw and a plate to reduce the need to bend the plate into different configurations, consequently minimizing the disadvantages stemming from such bending.

Another object of the present invention is to provide spinal instrumentation that allows the screw to be oriented relative to the plate over a wide range of angles, and locked into a particular angular orientation by means of a locking device.

### SUMMARY OF THE INVENTION

Further objects, forms, embodiments, aspects, benefits, features, and advantages of the invention will be clear from the following description with reference to the accompanying drawings which illustrate several embodiments of the invention by way of non-limiting examples.

In accordance with the present invention, spinal osteosynthesis instrumentation comprises the features of claim 1.

Other features and advantages of the present invention will become apparent in the course of the following description, given with reference to the accompanying drawings which show various embodiments of the invention by way of non-limiting examples.

### BRIEF DESCRIPTION OF THE DRAWINGS

Figure 1 is a top perspective view of one embodiment of a plate-type spinal osteosynthesis instrumentation in accordance with one aspect of the present invention.
Figure 2 is a bottom perspective view of the instrumentation of Figure 1.
Figure 3 is a top perspective view, similar to Figure 1, of another embodiment of instrumentation in which the spinal plates are provided with a ball and a cup disposed at opposite ends thereof to constitute a module that can be articulated relative to another adjacent module of similar construction.
Figure 4 is a partial top perspective view of another embodiment of instrumentation in accordance with another aspect of the present invention.
Figure 5 is a partial bottom perspective view of another embodiment of instrumentation in accordance with another aspect of the present invention.
Figure 6 is a top perspective view of the instrumentation of Figure 5.
Figure 7 is a partial top perspective view of another embodiment of instrumentation in accordance with another aspect of the present invention.
Figure 8 is a partial bottom perspective view of the instrumentation of Figure 7.
Figure 9 is a top perspective view of another embodiment of instrumentation in accordance with the present invention.
Figure 10 is a side perspective view of another embodiment of instrumentation in accordance with another aspect of the present invention.
Figure 11 is a partial cross-section and front view of the instrumentation of Figure 10.

### DESCRIPTION OF SELECTED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is hereby intended, such alterations and further modifications in the illustrated device, and such further applications of the principles of the invention as illustrated herein being contemplated as would normally occur to one skilled in the art to which the invention relates.

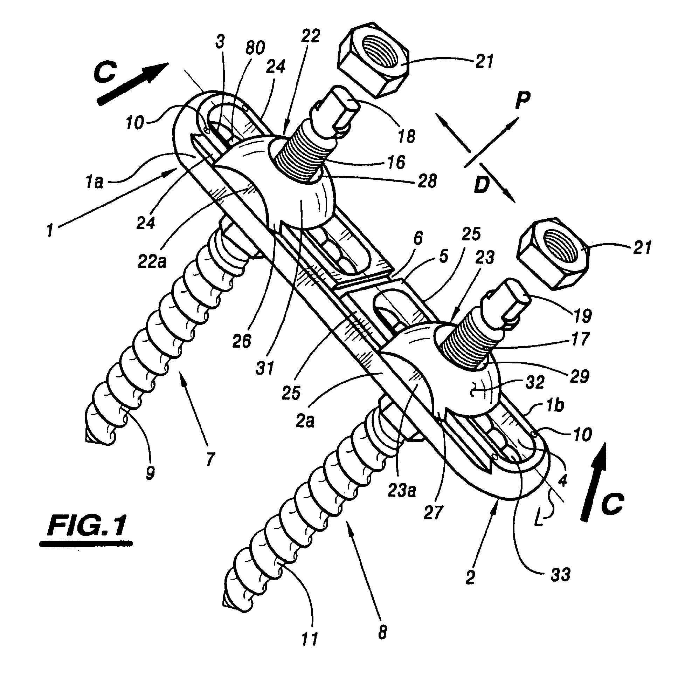

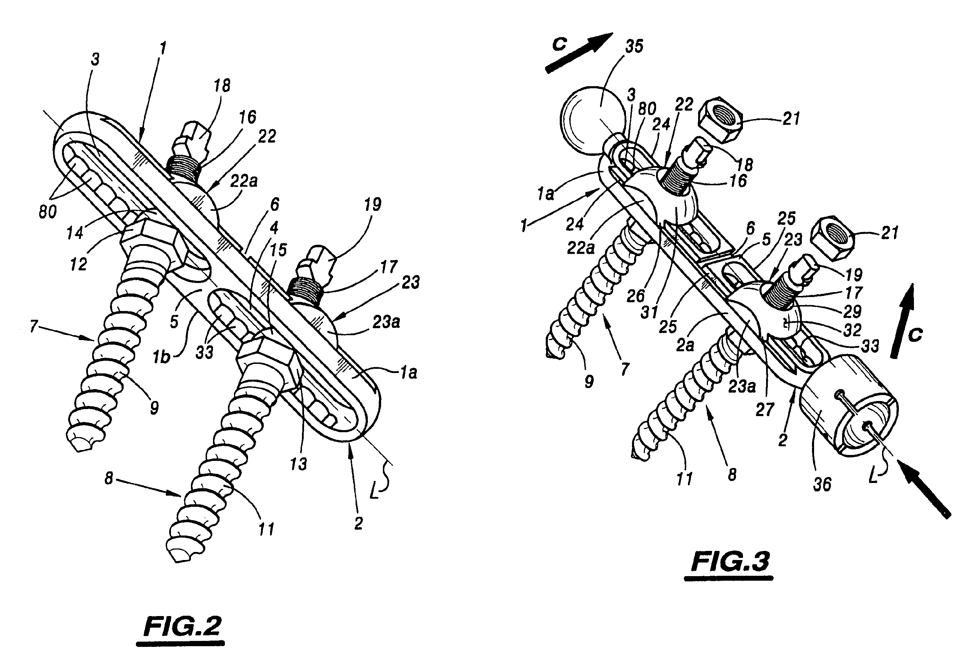

The spinal osteosynthesis instrumentation shown in Figures 1 and 2 comprises a pair of elongate vertebral plates 1 and 2 disposed end-to-end along a longitudinal axis L and positionable adjacent the spine. In the illustrated embodiment, plates 1 and 2 are integrally formed to define a single unitary piece. However, it should be understood that plates 1, 2 could be formed separately and interconnected by any method known to those of skill in the art, such as, for example, by fastening or welding. Each of the plates 1, 2 includes an oblong longitudinal opening 3, 4. The openings 3, 4 are separated by an intermediate junction area 5 in which is formed a transverse notch 6 constituting a weakened area. The notch 6 is intended to facilitate bending of the plates 1, 2, for example, in the direction of arrows C. Each plate 1, 2 is adapted to accept a bone anchor 7, 8 configured to be anchored in a vertebral body (not shown) to which the instrumentation is fitted.

Each bone anchor 7, 8, which in the illustrated embodiment is a bone screw, comprises a threaded shank 9, 11 configured to engage a vertebral body, a head 12, 13 having a polygonal portion, preferably contiguous with the threaded shank 9, 11 and adapted to drive the bone screw, a ball extending from the head 12, 13 and having a spherical bearing surface 14, 15, and a threaded end portion 16, 17 extending from the spherical bearing surface 14, 15. Each of the threaded end portions 16, 17 includes a smooth end portion 18, 19 for facilitating the guiding of a locking member, such as locking nut 21, along end portions 16, 17. Nut 21 is adapted to be screwed onto a respective threaded end portions 16, 17. Each threaded end 16, 17 is adapted to be inserted through a corresponding oblong opening 3, 4, and the associated locking nut 21 is adapted to lock the screw 7, 8 relative to the plate 1, 2 in a specific longitudinal position and angular orientation within the opening 3, 4.

The instrumentation further comprises, for each plate 1, 2, a respective connecting member or shoe 22, 23 mounted to slide on shaped grooves or slides 24, 26 formed along at least a portion of the length of the plates 1, 2 and configured to interconnect plates 1, 2 and screws 7, 8. In the illustrated embodiment, the shoes 22, 23 are mounted on the same side of the plates 1, 2 as the locking nuts 21 are disposed on. However, it should be understood that the shoes 22, 23 can alternatively be mounted on the opposite side of plates 1, 2. Each pair of slides 24, 25 is formed adjacent opposite longitudinal sides of the respective oblong opening 3, 4 and has a suitable profile to allow the shoe 22, 23 to slide along a longitudinal direction D while positively retaining the shoe 22, 23 on the plate 1, 2 in a transverse direction P. Thus, shoe 22, 23 is maintained or captured on a face of the plate 1, 2 in a transverse direction P, preferably substantially perpendicular to the direction of sliding D, without the aid of additional components, such as nuts or other types of fasteners. In the embodiment illustrated in Figures 1 and 2, the slides 24, 25 and the conjugate edges or shaped projections 26, 27 of the shoes 22, 23 have a male-female dovetail configuration. The shaped projections 26, 27 of the shoes 22, 23 are slidably engaged within the shaped grooves 24, 25 of plate 1, 2 to allow the shoes 22, 23 to slide relative to the plates 1, 2 along the longitudinal axis L. However, other suitable profiles are also contemplated as would occur to one of ordinary skill in the art.

A passage or bore 28, 29 is defined through each shoe 22, 23, through which extends the threaded end portion 16, 17 of the screw 7, 8, respectively. Preferably, each bore 28, 29 has a diameter sized larger than that of the threaded end portion 16, 17 of the screw 7, 8 to allow a certain degree of angular movement of the screw 7, 8 relative to shoe 22, 23. In the illustrated embodiment, the bore 28, 29 is cylindrical; however, bore 28, 29 can also take on other shapes, such as, for example, a conical or circular shape. Alternatively, the bore 28, 29 can be oblong-shaped, having a length extending in a direction substantially parallel to the longitudinal direction D of the oblong openings 3, 4 to allow angular movement of the screws 7, 8 in the direction of sliding. Each shoe 22, 23 defines around its bore 28, 29 an outer coaxial spherical bearing surface 31, 32, configured substantially complementary to a corresponding inner spherical bearing surface (not shown) defined on the inner face of the nut 21. The nut 21 can therefore be engaged tightly against the spherical bearing surface 31, 32 when nut 21 is tightened onto threaded end portions 16, 17 to retain the screw 7, 8 in a particular angular orientation relative to shoe 22, 23. The shoes 22, 23 are preferably mounted on the face of the plate 1, 2 on the same side as the nut 21, and have lateral flats 22a, 23a generally lying in the same plane as the lateral edges 1 a, 2a of the plates 1, 2.

The shaped grooves on slides 24, 25 extend substantially the entire length of the plates 1, 2, thus allowing the shoes 22, 23, and consequently the screws 7, 8 that pass through shoes 22, 23, to be variably adjusted in the direction of sliding D over virtually the entire length of the openings 3, 4. Formed on a lower portion of the longitudinally extending inside walls of oblong openings 3, 4 are concave spherical recesses or housings 80, 33 configured substantially complementary to the convex spherical bearing surfaces 14, 15 of screws 7, 8. The spherical bearing surfaces 14, 15 are configured to bear against the spherical housings 80, 33 to aid in maintaining the longitudinal position and angular orientation of screws 7, 8 relative to the plates 1, 2. It should be understood that the spherical housings 80, 33 could alternatively be replaced with conical housings to inhibit the axial sliding of screws 7, 8 when the nut 21 is tightly engaged against the shoe 22, 23. Abutments are provided on opposite ends of the slides 24, 25 along the longitudinal axis L to prevent the shoe 22, 23 from sliding off of slides 24, 25 and disengaging plates 1, 2. In the illustrated embodiment, stamped bosses 10 are provided at the ends of plates 1, 2 to maintain the shoes 22, 23 in sliding engagement with the plates 1, 2. In one embodiment, the stamped bosses 10 are formed in plates 1, 2 by punching or embossing.

The use of the spinal instrumentation described above is substantially as follows. It should be understood, however, that the following description of use is exemplary, and that other methods of using the instrumentation are also contemplated as being within the scope of the invention. The surgeon first drives the pedicular screws 7, 8 into the vertebral bodies by way of the hexagonal portions 12, 13, orienting the screws 7, 8 as close as possible to the appropriate angular orientation. The surgeon then inserts the threaded end portions 16, 17 of the screws 7, 8 into the bores 28, 29 of the shoes 22, 23, orienting the plates 1, 2 as required. The appropriate position of the screws 7, 8 along the sagittal plane of the patient (i.e., along the direction of sliding D) is obtained by virtue of the shoes 22, 23. The shoes 22, 23 can be slid along the plate 1, 2 in the direction of sliding D to accommodate a range of positions of screws 7, 8 within oblong openings 3, 4, with the spherical bearing surfaces 14, 15 being positioned in a corresponding spherical housings 80, 33. If the cylindrical bores 28, 29 are replaced by oblong bores, the surgeon has an additional means for adjusting the position and orientation of screws 7, 8 along the sagittal plane.

When the relative positions and orientations of the plates 1, 2 and the pedicular screws 7, 8 have been selected, the surgeon engages the nuts 21 onto the threaded end portions 16, 17. The smooth end portions 18, 19 define lateral flats which facilitate the guiding of the nuts 21 onto the screws 7, 8 and the initial threading of the nuts 21 onto the threaded end portions 16, 17. Without lateral flats 18, 19, it would be more difficult for the surgeon to thread the nuts 21 onto the screws 7, 8. As the nuts 21 are threaded along end portions 16, 17, the inner spherical bearing surfaces (not shown) of the nuts 21 are firmly engaged against the outer spherical bearing surfaces 31, 32 of the shoes 22, 23. Preferably, the inner and outer bearing surfaces have the same radius of curvature, thus having the effect of automatically centering the shoes 22, 23 relative to the screws 7, 8 when the nuts 21 are tightened due to the geometrical match between the complementary male and female bearing surfaces. The connection between the plates 1, 2 and the screws 7, 8, by virtue of the shoes 22, 23, allows for variable positioning and angular movement therebetween, with the screws being affixed to the plates in a particular position and at a particular angular orientation by the locking nut 21. More particularly, the tightening of nuts 21 onto the upper surfaces 31, 32 of shoes 22, 23 firmly engages the shoes 22, 23 against the plates 1, 2, and also firmly engages the spherical bearing surfaces 14, 15 of screws 7, 8 against the spherical recesses 80, 33.

Depending on the specific geometry of the screws 7, 8, there can be one shoe 22, 23 associated with each plate 1, 2 and oblong opening 3, 4, or alternatively two shoes 22, 23 if the openings 3, 4 are of a sufficient length. Additionally, asperities or surface roughness can be formed along at least a portion of the length of the slides 24, 25 and/or along the facing surfaces of the edges 26, 27 of the shoes 22, 23 to create resistance to sliding of the shoe 22, 23 in the direction of sliding D. The respective asperities must be of concordant or complementary form and must be limited in depth to allow for the free sliding of the shoes 22, 23 along the plates 1, 2 when the nuts 21 are not firmly engaged against the shoes 22, 23.

In Figures 1 and 2, the plates 1, 2 are illustrated in an unbent configuration, and therefore have a substantially rectilinear configuration. However, the transverse notch 6 between plates 1, 2 defines a weakened area which facilitates the bending of the single-piece plate 1, 2 along the direction of sliding D. The weakened area is preferably restricted to the location of the junction area or bridge of material 5 between the plates 1, 2 in order to maintain strength elsewhere along the length of the plates 1, 2. The bending of the plates 1, 2 along the sagittal plane of the patient, if required, is likely to be limited (for example, approximately 5º) because of the possibility of angulation of the screws 7, 8 relative to the plates 1, 2. The bending area is localized and is positioned intermediate the oblong openings 3, 4 to prevent damage to other functional parts of plates 1, 2, such as the slides 24, 25.

Because the above-described instrumentation allows for variable longitudinal and angular positioning of screws 7, 8 relative to plates 1, 2 and the selective fixation thereof, all of the usual spinal correction maneuvers and procedures are possible; such as, for example, compression, traction, and adjustment along the sagittal plane (lordosis).

Another embodiment of instrumentation in accordance with the invention is illustrated in Figure 3. The embodiment illustrated in Figure 3 differs from the previous one illustrated in Figures 1 and 2 in that the plates 1, 2 are equipped at their opposite ends with connecting means 35, 36 that allow for articulation relative to other adjacent plates provided with similar connecting means. More particularly, in the embodiment of the invention illustrated in Figure 3, the end of plate 1 is provided with a ball 35, and the opposite end of plate 2 is equipped with a cup 36. Preferably, the ball 35 and the cup 36 are formed integral with the plates 1, 2; however, it should be understood that ball 35 and cup 36 may be formed separate from plates 1, 2 and attached thereto by any method known to those of skill in the art. Ball 35 is configured to be accommodated or received in a cup 36 of an adjacent plate (not shown). In this manner, adjacent plates can be connected together to form a string of plates that can be articulated relative to one another. Alternatively, the plates 1, 2 could be provided with a ball 35 only or a cup 36 only, in which case only one end of the module could be articulated relative to another adjacent module. In another variant, the instrumentation may consist of a single plate 1 or 2 configured to accept at least one pedicular screw 7, 8. A short plate of this kind could be provided with a ball 35 only, or a cup 36 only, or both a ball 35 and a cup 36 to allow for articulation relative to an adjacent module.

Vertebral plates 1, 2 equipped with a ball 35 and/or a cup 36 constitute a module that can be variably articulated relative to other adjacent modules in three dimensions. The modules may then be rigidly fixed or locked in a selected orientation relative to adjacent modules by appropriate means, such as, for example, by shape-memory alloy clamping rings (not shown) disposed about the exterior of cups 36. Shape-memory rings of an appropriate type are described in French Patent Application 97 10 722 filed on 27 August 1997, the contents of which are hereby incorporated by reference. The ball 35 and the cup 36 assure a retentive articulation between adjacent modules, each module including a plate 1, 2 equipped with at least one pedicular screw 7, 8 and at least one shoe 22, 23. Such articulation allows for variable orientation of the modules relative to adjacent modules in all three dimensions, thus assuring a close fit of the resulting articulated chain of modules to the anatomy of a chosen section of the spine. The variable articulation of adjacent modules reduces the amount of bending which may otherwise be required if a single, one-piece plate were used to span several vertebral levels.

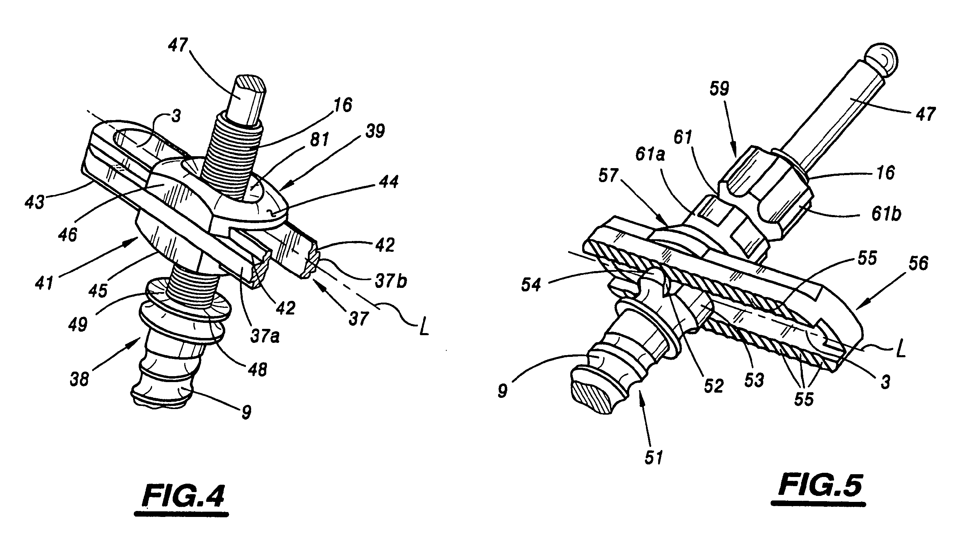

In another embodiment of the invention shown in Figure 4, the spinal instrumentation comprises a single, unbent vertebral plate 37 extending along a longitudinal axis L and defining a longitudinal opening 3, a bone anchor screw 38 and two shoes 39, 41 disposed on opposite sides of plate 37. The shoes 39, 41 are configured to slide along upper and lower sides of plate 37 by means of respective pairs of upper and lower longitudinal slides 42, 43. Each shoe 39, 41 has a central oblong bore 81 (only one of which can be seen in Figure 4) which is oriented substantially perpendicular to the longitudinal axis L and the oblong opening 3. The length of oblong bore 81 extends substantially along the sagittal plane of the patient when the instrumentation is fitted to a particular spinal segment (not shown). Each shoe 39, 41 also defines around its bore 81 an outer convex spherical bearing surface 44, 45, respectively, limited laterally by a pair of opposing flats 46 extending in the same plane as the opposing lateral faces 37a, 37b of plate 37.

The spherical bearing surface 44 of the upper shoe 39 cooperates with a locking nut configured similar to nut 21 (Figures 1 and 2). The threaded end portion 16 of the screw 38 includes a smooth end part 47 for facilitating the guiding of the nut 21 onto the threaded end portion 16. The threaded shank 9 of the screw 38 is extended by a flange 48, delimiting a concave spherical annular bearing surface 49 corresponding to the convex spherical bearing surface 45 of lower shoe 41. The concave spherical bearing surface 49 of the screw 38 is extended by the threaded end portion 16. The threaded end portion 16 is inserted through the openings 81 in the upper and lower shoes 39, 41, and when the screw 38 is positioned in a desired position along oblong opening 3 and at a desired angular orientation relative to plate 37, nut 21 is tightened against the upper shoe 39. The tightening of the nut 21 onto the threaded end portion 16 causes the convex bearing surface 45 to be pressed tightly against the concave bearing surface 49 of the screw 38. Additionally, the nut 21 bears on the convex bearing surface 44 and locks the screw 38 in a particular angular orientation relative to plate 37. Tightening the nut 21 onto the threaded end portion 16 also causes the shoes 39, 41 to be compressed tightly against the slides 42, 43, thereby locking the shoes 39, 41 and the screw 38 in a particular longitudinal position along oblong opening 3. As in the previously described embodiments, tightening the nut 21 onto the upper shoe 39 automatically centers the shoes 39, 41 relative to the screw 38 and positions the shoes 39, 41 in the correct longitudinal position relative to plate 37.

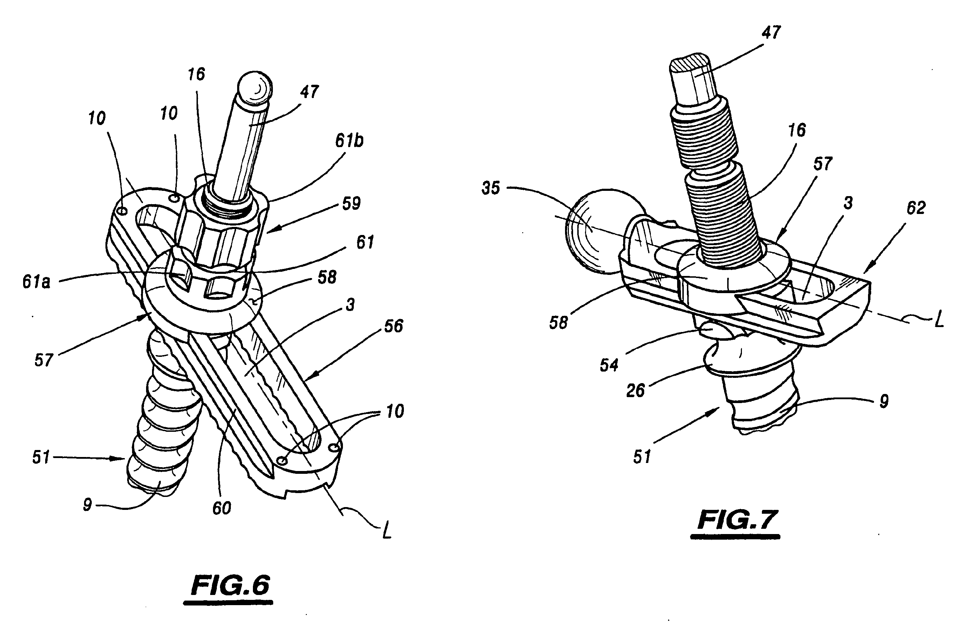

In another embodiment of the invention shown in Figures 5 and 6, the screw 51 includes a pair of opposite shoulders 54 projecting laterally outward from a head 53. Shoulders 54 each define a substantially cylindrical bearing surface 52. The cylindrical bearing surfaces 52 are adapted to cooperate with substantially complementary cylindrical-shaped surfaces or grooves 55 machined onto a longitudinally extending face of the plate 56. In one embodiment, cylindrical bearing surfaces 52 are convex and cylindrical bearing surfaces 55 are concave; however, a reverse embodiment is also contemplated as being within the scope of the invention. Cylindrical bearing surfaces 52 are configured to bear against cylindrical bearing surfaces 55 to allow angulation of the screw 51 relative to plate 56 in a direction substantially parallel with the longitudinal axis L and to aid in retaining the screw 51 in a selected longitudinal position along oblong opening 3. Preferably, the cylindrical surfaces 55 and the corresponding bearing surfaces 52 have axes extending substantially perpendicular to the longitudinal axis L of the plate 56. In this manner, the angulation of screw 51 relative to plate 56 will be limited to a plane oriented substantially parallel with longitudinal axis L.

On the face of the plate 56 opposite the cylindrical bearing surfaces 55 is disposed a shoe 57, mounted to slide on lateral slides 60 disposed along the opposing walls of the opening 3. As in previously described embodiments of the invention, the shoe 57 has a spherical bearing surface 58 adapted to cooperate with a substantially complementary spherical bearing surface (not visible in Figures 5 and 6) of a locking nut 59 that is configured to engage the threaded end portion 16 of screw 51. As illustrated, the nut 59 can be of a type having a middle area 61 which breaks or fractures upon application of a predetermined tightening torque, thereby creating a first portion 61 a that remains tightly engaged against the shoe 57, and a second portion 61 b that can be discarded after the nut fractures along the weakened area 61. A "self-breaking" nut of the above type is described in French Patent 94 10 377 (2 723 837), the contents of which are hereby incorporated by referenced into the subject application.

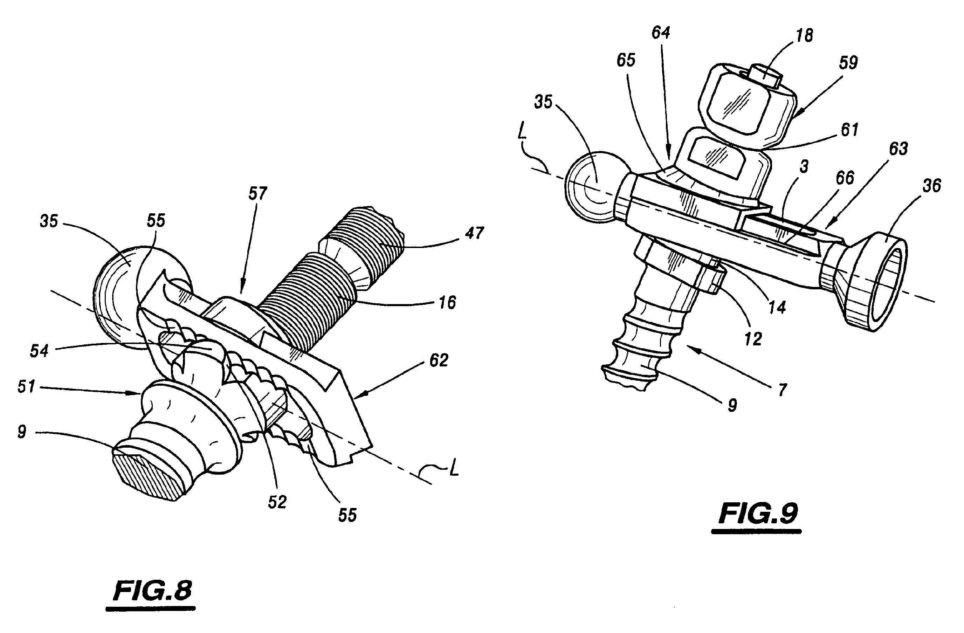

Another embodiment of instrumentation in accordance with the present invention is illustrated in Figures 7 and 8. A plate 62 differs from the plate 56 illustrated in Figures 5 and 6 in that it is equipped at one end with a ball 35 configured to be inserted into a cup 36 of a second adjacent vertebral plate (not shown). The second plate can be identical to any of the plates of the embodiments described above, including the plates 1, 2, with one end of the second plate being equipped with a cup 36 for articulating connection with the ball 35 on plate 62 to thereby form a string of articulating modules.

Figure 9 illustrates yet another embodiment of the present invention, comprising a plate 63 equipped at its opposite ends with a ball 35 and a cup 36. In one embodiment, the ball 35 and cup 36 are integrally formed with plate 63. The elongate opening 3 is straddled by a shoe 64 having a spherical bearing surface 65, on which bears a self-breaking nut 59. The shoe 64 is mounted to slide in slides 66 disposed on opposite sides of the opening 3 along the longitudinal axis L. A pedicular screw 7 is engaged in the shoe 64 in a manner similar to that described above with regard to other embodiments of the invention. Plate 63 may be articulated relative to other similarly configured plates by way of cooperation between adjacent balls 35 and cups 36 in a manner similar to that described above with regard to other embodiments of the invention. Thus, a plurality of plates 63 can be connected to each other in particular orientations and thereafter made rigid, as previously indicated, by using, for example, shape memory alloy rings. Of course, it is also possible to provide plates, such as plate 62, with cups 36 complementary to balls 35 to enable construction of a string of modules having retentive articulations, with each module oriented in a particular orientation relative to adjacent modules in all three dimensions.

In all of the embodiments of the invention illustrated and discussed above, the diameter or width of the openings (28, 29, ...) extending through the shoes (22, 23, ...) is greater than the diameter of the threaded end portion 16 of the screws (7, 8, ...). This feature, coupled with the interaction between the spherical bearing surfaces of the shoes and the nuts (21, 59, ...), allow the angular orientation of the screws (7, 8 ...) to be varied in all three directions relative to the plate (1, 2, ...).

It should be understood that it is possible to produce in the context of the invention instrumentation consisting of plates 1, 2, 37, 56, 62, provided with or without balls 35 and/or cups 36. Also, each plate (1, 2 ...) can be equipped with one or more pedicular screws, and with a corresponding number of shoes configured to slide along the longitudinal opening 3. Additionally, each screw can be associated with a single sliding shoe disposed on one side of the plate, or with two shoes disposed on opposite sides of the plate.

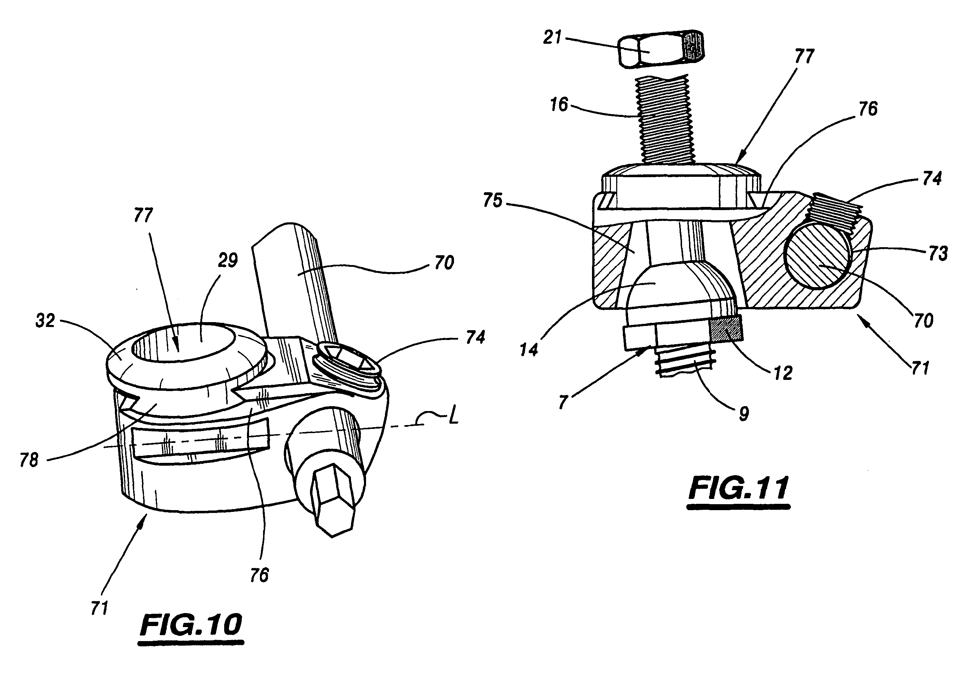

Another embodiment of the present invention is illustrated in Figures 10 and 11, comprising a vertebral rod 70 and a connector 71 transversely connecting rod 70 to a pedicular screw 7. The connector 71 defines a bore 73 sized to receive rod 70 therethrough. Rod 70 can be locked into position by means of a threaded plug or set screw 74 engaged within a threaded hole intersecting the bore 73. Connector 71 also defines a longitudinal axis L and an oblong opening 75 extending therethrough and being elongated in the direction of the longitudinal axis L and transverse to the rod 70. The internal walls of the opening 75 are preferably inclined so as to define a conical surface; however, other configurations of opening 75 are also contemplated, such as a cylindrical configuration. Along each side of the opening 75, slides 76 are formed. A shoe 77 is slidably mounted to the slides 76 so as to allow the shoe 77 to freely glide in a direction along longitudinal axis L and transverse to rod 70. The shoe 77 is configured similar to shoes 22, 23 illustrated and discussed above. Shoe 77 can alternatively be configured similar to the shoes associated with any of the preceding embodiments of the invention, with the connector 71 being correlatively adapted.

In the embodiment of Figures 10 and 11, shoe 77 has dovetail-shaped edges or projections 78, slidably received within complementary mortises or grooves which form slides 76. Thus, the shoe 77 can slide along a certain length of the connector 71 in a direction transverse to the rod 70, while also allowing angulation of screw 7 relative to the connector 71. The convex spherical bearing surface 32 of shoe 77 cooperates with a corresponding concave bearing surface (not shown) formed on nut 21. The screw 7 is locked at a particular angle relative to the shoe 32, and the shoe 32 is locked in a particular longitudinal position relative to connector 71, by tightening the nut 21 onto the spherical bearing surface 32. The connector 71 is itself fixed to the rod 70 by tightening the set screw 74 against an outer surface of rod 70.

The screws, plates, shoes and other components of the present invention are preferably made from a bio-compatible material of suitable strength, such as, for example, stainless steel, titanium, or other compositions as would occur to one of ordinary skill in the art.

All publications, patents, and patent applications cited in this specification are herein incorporated by reference as if each individual publication, patent, or patent application were specifically and individually indicated to be incorporated by reference and set forth in its entirety herein. Any theory of operation or finding described herein is merely intended to enhance understanding of the present invention and should not be construed to limit the scope of the present invention as defined by the claims that follow to any stated theory or finding.

While the invention has been illustrated and described in detail in the drawings and foregoing description, the same is to be considered as illustrative and not restrictive in character, it being understood that only the preferred embodiment has been shown and described and that all changes, modifications, and equivalents that come within the scope of the invention as defined by the following claims are desired to be protected. For example, the features disclosed in reference to the embodiments of the invention illustrated in Figures 1-9 and discussed in detail above, and in particular the provision of the two shoes slidably mounted on opposite faces of the plate, may be incorporated into the embodiment of the invention illustrated in Figures 10-11.

## Claims

1. A device for spinal osteosynthesis, comprising :
a first member (1, 2, 7, 71...) having a longitudinal axis (L) and being positionable adjacent the spine ;
a second member (7, 8) having a first end portion (9, 11) configured to engage a vertebral body and a second end portion (16, 17);
a connector member (22, 23, 39, 57, 77) interconnecting said second end portion (16, 17) of said second member (7, 8) with said first member (1, 2, 37, 71...) in a variable position along said longitudinal axis (L), said connector member (22, 23, 39, 57, 77) being positively retained relative to said first member (1, 2, 37, 71 ...) in a direction (P) substantially perpendicular to said longitudinal axis (L), one of said first member (1, 2, 37, 71...) and said connector member (22, 23, 39, 57, 77) including at least one shaped groove (24, 25, 42, 60, 76) extending along said longitudinal axis (L) and another of said first member (1, 2, 37, 71...) and said connector member (22, 23, 39, 57, 77) including at least one shaped projection (26, 27, 78) configured substantially complementary to said shaped groove (24, 25, 42, 60, 76), said shaped projection, (26, 27, 78) being slidably engaged within said shaped groove (24, 25, 42, 60, 76) **characterized in that** said shaped projection and said shaped groove allow said connector member (22, 23, 39, 57, 77) to slide relative to said first member (1, 2, 37, 71...) in a direction along said longitudinal axis (L), and cooperates to allow said connector member (22, 23, 39, 57, 77) to slide along a longitudinal face of said first member (1, 2, 37, 71...) while being captured on said first member (1, 2, 37, 71...) in a direction (P) substantially perpendicular to said longitudinal axis (L) and **in that** a locking member (21, 59) cooperates with said connector member (22, 23, 39, 57, 77) and said second end portion (16, 17) to lock said second end portion (16, 17) in a particular position along said longitudinal axis (L).

2. Device according to claim 1, **characterized in that** it has a pair of shaped grooves (24, 25, 42, 60, 76) and a pair of shaped projections (26, 27, 78).

3. Device according to claim 1, wherein said connector members (22, 23, 39, 57, 77) has a passage (28, 29) extending therethrough and being configured to receive said second end portion (16, 17) of said second member (7, 8) therein at a variable angle relative to said first member (1, 2, 37, 71...), said passage (28, 29) opening onto a first substantially spherical-shaped bearing surface, said locking member (21, 59) defining a second substantially spherical-shaped bearing surface substantially complementary to said first spherical-shaped bearing surface, said locking member (21, 59) cooperating with said connector member (22, 23, 39, 57, 77) and said second end portion (16, 17) to tightly engage said first and second spherical bearing surfaces and lock said second end portion (16, 17) at a particular angle relative to said first member (1, 2, 37, 71...).

4. Device according to claim 3, wherein said passage (28, 29) is cylindrical-shaped.

5. Device according to claim 3, wherein said passage (28, 29) is conical-shaped.

6. Device according to claim 1, wherein said first member defines a pair of abutments disposed along said longitudinal axis (L) on opposite sides of said connector member (22, 23, 39, 57) for maintaining said connector member (22, 23, 39, 57) in sliding engagement with said first member (1, 2, 37).

7. Device according to claim 6, wherein said abutments (10) are pressed bosses (10).

8. Device according to claim 1, wherein said shaped groove (24, 24, 42, 60) and said shaped projection (26, 27, 28) define asperities to resist sliding of said connector member (22, 23, 39, 57), relative to said first member (1, 2, 37, 71...).

9. Device according to claim 1, wherein said at least one shaped groove (24, 25, 42, 60, 76) and said at least one projection (26, 27, 78) have a dovetail configuration.

10. Device according to claim 1, wherein said first member (1, 2, 37, 71...) defines an oblong opening (3, 4, 75) extending therethrough and having a length extending along said longitudinal axis (L), said connector member (22, 23, 39, 57, 77) defining a passage (28, 29) extending therethrough, said second end portion (16, 17) of said second member (7,8) passing through said oblong opening (3, 4, 75) and said passage (28, 29) and being positionable along said length of said oblong opening (3, 4, 75) in an infinite number of positions, a portion of said second end portion (16, 17) extending from said connector (22, 23, 39, 57, 77), said locking member (21, 59) engaging said portion of said second end portion (16, 17) to lock said second end portion (16, 17) in a particular position along said length of said oblong opening (3, 4, 75).

11. Device according to claim 10, wherein said first member (1, 2, 37, 71,...) defines pair of slides (24, 25, 42, 60, 76) extending along said longitudinal axis, each of said slides (24, 25, 42, 60, 76) being disposed on opposite sides of said opening (3, 4, 75) said connector member (22, 23, 39, 57, 77) defining a pair of lateral edges (26, 27, 78), each of said lateral edges (26, 27, 78) having a profile substantially complementary to a corresponding one of said slides (24, 25, 42, 60, 76), said pair of lateral edges (26, 27, 78) being engaged with said pair of slides (24, 25, 42, 60, 76) to allow said connector member (22, 23, 39, 57, 77) to slide relative to said first member (1, 2, 37, 71, ...) in a direction along said longitudinal axis (L).

12. Device according to claim 11, wherein said pair of lateral edges (26, 27, 78) are engaged with said pair of slides (24, 25, 42, 60, 76) in a dovetail configuration, said dovetail configuration allowing said connector member (22, 23, 39, 57, 77) to slide relative to said first member (1, 2, 37, 71, ...) in said direction along said longitudinal axis (L) while capturing said connector member (22, 23, 39, 57, 77) on a longitudinally extending face of said first member (1, 2, 37, 71...).

13. Device according to claim 10, wherein said passage (28, 29) of said connector member (22, 23, 39, 57, 77) is configured to receive said second end portion (16, 17) of said second member (7, 8) at variable angle relative to said first member (1, 2, 37, 71...), said passage (28, 29) opening onto a first substantially spherical-shaped bearing surface, said locking member (21, 59) defining a second substantially spherical-shaped bearing surface substantially complementary to said first substantially spherical-shaped bearing surface, said locking member (21, 59) engaging said portion of said second end portion (16, 17) and tightly compressing said first and second substantially spherical bearing surfaces to lock said second end portion (16, 17) at a particular angle relative to said first member (1, 2, 37, 71...).

14. Device according to claim 10, wherein said portion of said second end portion (16, 17) is threaded and said locking member (21, 59) is a lock nut.

15. Device according to claim 14, wherein said lock nut (59) includes a break-off portion (61 b) that is separated from the remaining portion (61a) of said lock nut (59) upon application of a predetermined amount of torque to said break-off portion (61b).

16. Device according to claim 10, wherein said second member (7, 8) has a shoulder (12, 13, 54) disposed between said first (9, 11) and second (16, 17) end portions, said shoulder (12, 13) facing a first side of said first member (1, 2, 37, 71,...) when said second end portion (16, 17) is passed through said oblong opening (3, 4, 75) said connector member (22, 23, 39, 57, 77) being disposed on an opposite second side of said first member (1, 2, 37, 71,...) said shoulder (12, 13, 54) being configured to bear against said first side of said first member (1, 2, 37, 71,...) to lock said second member (7, 8) in said particular position along said length of said oblong opening (3, 4, 75).

17. Device according to claim 10, further comprising another of said connector members (22, 23, 39, 57) positively retained on an opposite side of said first member (1, 2, 37, ...) in a direction (P) substantially perpendicular to said longitudinal axis (L), said second end portion (16, 17) of said second member (7, 8) passing through said oblong opening (3, 4) and each of said passages (28, 29) in said pair of connector members (22, 23, 39, 57) and being variably positionable along said length of said oblong opening (3, 4) said locking member (21, 59) engaging said portion of said second end portion (16, 17) to lock said second end portion (16, 17) in said particular position along said length of said oblong opening (3, 4).

18. Device according to claim 1, wherein said first member is an elongate plate (1, 2, 37, 56, 62, 63) having a length extending along said longitudinal axis (L) and being positionable along the spine.

19. Device according to claim 1, further comprising an elongate rod member (70) positionable along the spine, said first member (71) defining an opening (73) extending therethrough in a direction transverse to said longitudinal axis (L) and being sized to receive said elongate member (70) therein to transversely connect said rod member (70) to said second member (7).

20. Device according to claim 1 further comprising a plurality of said first members (1, 2, 37), said plurality of first members (1, 2, 37) being integrally formed to define a single piece extending along said longitudinal axis (L), said single piece defining a weakened area (6) intermediate adjacent ones of said plurality of said first members (1, 2, 37) to facilitate bending of said single piece along said longitudinal axis, each of said plurality of first members (1, 2 37) being associated with corresponding ones of said second member (7, 8) said connector member (22, 23, 39, 57) and said locking member (21).

21. Device according to claim 20, wherein said weakened area comprises a notch (6) extending across said single piece in a direction substantially perpendicular to said longitudinal axis (L).

22. Device according to claim 21, wherein said notch (6) is disposed on the same side of said single piece as said locking member (21).

23. Device according to claim 1, wherein said first member (1, 2) defines upper and lower surfaces and a pair of laterally facing sides (1a, 2a) extending therebetween, said connector member (22, 23) being disposed adjacent said upper surface and including a pair of laterally facing flattened portions (22a, 23a) lying substantially in the same plane as said pair of laterally facing sides (1a, 2a).

24. Device according to claim 1, wherein said first member (1, 2, 37, 71...) is positionable along the spine with said longitudinal axis (L) being oriented substantially parallel with a sagittal plane.

25. Device according to claim 1, further comprising at least two of said first members (1, 2, 62, 63) disposed end to end along said longitudinal axis (L), one (1, 62) of said first members including a ball portion (35) and another adjacent one (2, 63) of said first members including a socket portion (36) configured to receive said ball portion (35) therein, said ball portion (35) being inserted within said socket portion (36) to provide relative articulation between said at least two of said first members (1, 2, 62, 63) in three dimensions.

26. Device according to claim 25, wherein each of said at least two of said first members (63) includes one of said ball portions (35) disposed adjacent one end thereof and one of said socket portions (36) disposed adjacent an opposite second end thereof.

27. Device according to claim 25, further comprising means for retaining said ball portion (35) in said socket portion (36) and maintaining said at least two of said first member (1, 2, 62, 63) in a particular orientation relative to one another.

28. Device according to claim 27, wherein said retaining means comprises a ring sized to receive said socket portion (36) therein and being at least partially formed of a shape-memory material.

29. Device according to claim 1, wherein said connector member (22, 23, 39 77) is positionable in an infinite number of positions along said longitudinal axis (L) of said first member (1, 2, 37, 71...).

30. Device according to claim 1, wherein said first member (1, 2, 37, 71....) has a first side and an opposite second side each extending along said longitudinal axis (L), said first member (1, 2, 37, 71) defining an opening (3, 4, 75) extending between said first and second sides, said connector member (22, 23, 39, 57, 77) defining a passage (28, 29) extending therethrough and being sized to receive said second end portion (16, 17) of said second member (7, 8) therein, said connector member (22, 23, 39, 57, 77) being disposed adjacent said first side with said first side with said passage (28, 29) being alignable with said opening (3, 4, 75) ; and
wherein said second member (7, 8) has a shoulder (12, 13, 54) disposed between said first (9, 11) and second (16. 17) end portions, said shoulder (12, 13, 54) being configured to bear against said second side of said first member (1, 2, 37, 71...) when said second end portion (16, 17) is passed through said opening (3, 4, 75) and said passage (28, 29) to aid in retaining said second end portion (16, 17) in said particular position along said longitudinal axis (L).

31. Device according to claim 30, wherein said shoulder (54) defines at least one cylindrical-shaped bearing surface (52) projecting laterally from said second member (7, 8), said second side of said first member (56, 62) defining a plurality of cylindrical-shaped bearing surfaces (55) substantially complementary to said at least one cylindrical-shaped bearing surface (52), said plurality of cylindrical-shaped bearing surfaces (52) being disposed along said longitudinal axis (L) adjacent said opening (3, 4) and extending in a direction (P) substantially perpendicular to said longitudinal axis (L) ; and
wherein said at least one cylindrical-shaped bearing surface (52) is configured to bear against a corresponding one of said plurality of cylindrical-shaped bearing surfaces (55) to aid in retaining said second end portion (16, 17) at said particular position, along said longitudinal axis (L) and to limit angulation of said second member (7, 8) relative to said first member (56, 62) in a plane oriented substantially parallel to said longitudinal axis (L).

32. Device according to claim 31, wherein said shoulder (54) defines a pair of said at least one cylindrical-shaped bearing surface (52) each extending from said second member (7, 8) in opposite directions, said first member (56, 62) defining corresponding pairs of said plurality of cylindrical-shaped bearing surfaces (55) each disposed along opposite sides of said opening (3, 4) ; and
wherein said pair of said at least one cylindrical-shaped bearing surface (52) is configured to bear against a respective one of said corresponding pairs of said plurality of cylindrical-shaped bearing surfaces (55) to aid in retaining said second end portion (16, 17) at said particular position along said longitudinal axis (L) and to limit angulation of said second member (7, 8) relative to said first member (56, 62) in a plane oriented substantially parallel to said longitudinal axis (L).

33. Device according to claim 31, wherein said at least one cylindrical-shaped bearing surface (52) is convex and said plurality of cylindrical-shaped bearing surfaces (55) are concave.

34. Device according to claim 30, wherein said shoulder (12, 13) defines a spherical-shaped bearing surface (14, 15) said first member (1, 2) defining a plurality of recesses (80, 33) substantially complementary to said spherical-shaped bearing surface (14, 15), said recesses (80, 33) being disposed along said opening (3, 4) adjacent said second side of said first member (1, 2), and
wherein said spherical-shaped bearing surface (14, 15) is configured to bear against a corresponding one of said plurality of recesses (80, 33) to aid in retaining said second end portion (16, 17) at said particular position along said longitudinal axis (L).

35. Device according to claim 1, further comprising another of said connector members (22, 23, 39), said first member (1, 2, 37) having opposite sides and defining an opening (3, 4) extending therebetween, said connector members (22, 23, 39) being disposed on said opposite sides of said first member (1, 2, 37), a first (22) of said connector members (22, 23) defining a first passage (28) extending therethrough and sized to receive said second end portion (16) of said second member (7) therein at a variable angle relative to said first member (1, 2, 37), said passage (28) opening onto a first substantially spherical-shaped bearing surface, a second (23) of said connector members (22. 23) defining a second passage (29) extending therethrough and sized to receive said second end portion (17) of said second member (8) therein at a variable angle relative to said first member (1, 2, 37), said second passage (29) opening onto a second substantially spherical-shaped bearing surface ;
said locking member (21) defining a third substantially spherical-shaped bearing surface substantially complementary to said first spherical-shaped bearing surface (31) of said first connector member (22) ;
said second member (7) having a shoulder disposed between said first (9) and second (16) end portions, said shoulder defining a forth substantially spherical-shaped bearing surface (14) substantially complementary to said second substantially spherical-shaped bearing surface of said second connector member ; and
said second end portion (16) of said second member (7) extending through said opening (3) and said first (28) and second (29) passages in said first (22) and second (23) connector members, said locking member (21) cooperating with said connector member (22, 23) and said second end portion (16) to tightly engage said first (31) and third substantially spherical bearing surfaces and said second and forth substantially spherical bearing surfaces to lock said second end portion (16) at said particular position along said longitudinal axis (L) and at a particular angle relative to said longitudinal axis (L).

36. Device according to claim 35, wherein said first (22) and second (23) connector members are positively retained on said first member (1, 2, 37) in a direction (P) substantially perpendicular to said longitudinal axis (L).

37. Device according to claim 1, wherein
said first member (1, 2, 37...) is a plate
said second member (7, 8) is a bone anchor
said connector member (22, 23, 39, 57, 77) is slidable along a longitudinally extending face of said plate (1, 2, 37...) to position said connector member (22, 23, 39, 57, 77) infinite number of positions along said longitudinal axis (L).

38. Device according to claim 37, wherein said connector (22, 23; 39, 57, 77) defines a pair of opposing laterally extending projections (26; 27, 78) disposed on opposite sides of said longitudinally extending face of said plate (1, 2; 37...) to maintain said connector member (22, 23, 39, 57, 77) in sliding engagement with said plate (1, 2, 37...).

39. Device according to claim 38, wherein said laterally extending projections (26, 27) of said connector member (22, 23) cooperate with substantially complementary laterally extending grooves (24, 25) defined in said plate (1, 2) to provide a dovetail-type mounting arrangement between said connector member (12, 23) and said plate (1, 2).

40. Device according to claim 37, wherein said connector member (22, 23, 39, 57, 77) and said plate (1, 2, 37...) include cooperating surface means for positioning said connector member (22, 23, 39, 57, 77) in said infinite number of positions along said longitudinal axis (L) while positively retaining said connector member (22, 23, 39, 57, 77) on said face of said plate (1, 2, 37...).

41. Device according to claim 37, wherein said bone anchor (7, 8) and said plate (1, 2, 37...) include means for pivoting said bone anchor (7, 8) through a range of angles relative to said longitudinal axis (L) of said plate (1, 2, 37...).

42. Device according to claim 37, further comprising means for articulately connecting a plurality of said plates (62, 63) end-to-end along said longitudinal axis (L).

43. Device according to claim 1, wherein
said first member (1, 2, 37, 71...) defines an oblong opening (3, 4, 75) having a length extending in a longitudinal direction (L),
said second member (7, 8) is a screw having a bone anchoring shank (9, 11) end an opposite end portion (16, 17), said bone anchoring shank (9, 11) being configured to engage a vertebral body, said opposite end portion (16, 17) being sized to pass through said oblong opening (3, 4, 75), and
said locking member (21) locks said screw (7, 8) with respect to said first member (1, 2, 37, 71...) in a said particular position along said longitudinal axis (L) and with a particular angular orientation relative to said longitudinal axis (L), said locking member (21) including at least one shoe (22, 23) configured to slide on corresponding slides (24, 25) provided on said first member (1, 2, 37, 71...) in said longitudinal direction (L), said at least one shoe (22, 23) defining a bore (28, 29) sized to receive said and portion (16, 17) of said screw (7, 8) therein, said end portion (16, 17) of said screw (7, 8) being configured to cooperate with said locking member (21) to lock said screw (7, 8) in said particular longitudinal position and with said particular angular orientation.

## Patentansprüche

1. Vorrichtung für die Wirbelsäulenosteosynthese, aufweisend:
ein erstes Element (1, 2, 7, 71, ...), das eine Längsachse (L) hat und benachbart zu der Wirbelsäule positionierbar ist,
ein zweites Element (7, 8), das einen ersten Endabschnitt (9, 11), der zum Eingreifen in einen Wirbelkörper gestaltet ist, und eine zweiten Endabschnitt aufweist,
ein Verbindungselement (22, 23, 39, 57, 77), das den zweiten Endabschnitt (16, 17) des zweiten Elements (7, 8) mit dem ersten Element (1, 2, 37, 71, ...) in einer variablen Position entlang der Längsachse (L) verbindet, wobei das Verbindungselement (22, 23, 39, 57, 77) relativ zu dem ersten Element (1, 2, 37, 71) formschlüssig in einer Richtung (P) im Wesentlichen senkrecht zu der Längsachse (L) gehalten wird, wobei eines von dem ersten Element (1, 2, 37, 71, ...) und dem Verbindungselement (22, 23, 39, 57, 77) mindestens eine geformte Nut (24, 25, 42, 60, 76) aufweist, die sich entlang der Längsachse (L) erstreckt, und wobei ein anderes von dem ersten Element (1, 2, 37, 71, ...) und dem Verbindungselement (22, 23, 39, 57, 77) mindestens einen geformten Vorsprung (26, 27, 78) aufweist, der im Wesentlichen komplementär zu der geformten Nut (24, 25, 42, 60, 76) gestaltet ist, wobei der geformte Vorsprung (26, 27, 78) gleitend in die geformte Nut (24, 25, 42, 60, 76) eingreift, **dadurch gekennzeichnet, dass** der geformte Vorsprung und die geformte Nut erlauben, dass das Verbindungselement (22, 23, 39, 57, 77) relativ zu dem ersten Element (1, 2, 37, 71, ...) in einer Richtung entlang der Längsachse (L) gleitet, und zusammenwirken, um zu erlauben, dass das Verbindungselement (22, 23, 39, 57, 77) entlang einer Längsfläche des ersten Elements (1, 2, 37, 71, ...) gleitet, während es in einer Richtung (P) im Wesentlichen senkrecht zu der Längsachse (L) auf dem ersten Element (1, 2, 37, 71, ...) gefangen ist, und dadurch, dass ein Verriegelungselement (21, 59) mit dem Verbindungselement (22, 23, 39, 57, 77) und dem zweiten Endabschnitt (16, 17) zusammenwirkt zum Verriegeln des zweiten Endabschnitts (16, 17) in einer bestimmten Position entlang der Längsachse (L).

2. Vorrichtung gemäß Anspruch 1, **dadurch gekennzeichnet, dass** sie ein Paar von geformten Nuten (24, 25, 42, 60, 76) und ein Paar von geformten Vorsprüngen (26, 27, 78) aufweist.

3. Vorrichtung gemäß Anspruch 1, wobei das Verbindungselement (22, 23, 39, 57, 77) einen sich durch es erstreckenden Durchgang (28, 29) aufweist, der zum Aufnehmen des zweiten Endabschnitts (16, 17) des zweiten Elements (7, 8) in einem variablen Winkel relativ zu dem ersten Element (1, 2, 37, 71, ...) gestaltet ist, wobei sich der Durchgang (28, 29) auf eine erste im Wesentlichen kugelförmig geformte Führungsfläche öffnet, wobei das Verriegelungselement (21, 59) eine zweite im Wesentlichen kugelförmig geformte Führungsfläche definiert, die im Wesentlichen komplementär zu der ersten kugelförmig geformten Führungsfläche ist, wobei das Verriegelungselement (21, 59) mit dem Verbindungselement (22, 23, 39, 57, 77) und dem zweiten Endabschnitt (16, 17) zum engen Ineinandergreifen der ersten und der zweiten kugelförmigen Führungsfläche und zum Verriegeln des zweiten Endabschnitts (16, 17) in einem bestimmten Winkel relativ zu dem ersten Element (1, 2, 37, 71, ...) zusammenwirkt.

4. Vorrichtung gemäß Anspruch 3, wobei der Durchgang (28, 29) zylinderförmig ist.

5. Vorrichtung gemäß Anspruch 3, wobei der Durchgang (28, 29) konisch geformt ist.

6. Vorrichtung gemäß Anspruch 1, wobei das erste Element ein Paar von Anschlägen definiert, die entlang der Längsachse (L) auf entgegengesetzten Seiten des Verbindungselements (22, 23, 39, 57) zum Halten des Verbindungselements (22, 23, 39, 57) in einem gleitenden Eingriff mit dem ersten Element (1, 2, 37) angeordnet sind.

7. Vorrichtung gemäß Anspruch 6, wobei die Anschläge (10) gepresste Ansätze (10) sind.

8. Vorrichtung gemäß Anspruch 1, wobei die geformte Nut (24, 25, 42, 60) und der geformte Vorsprung (26, 27, 28) Oberflächen-Unebenheiten aufweisen, um dem Gleiten des Verbindungselements (22, 23, 39, 57) relativ zu dem ersten Element (1, 2, 37, 71) einen Widerstand entgegenzusetzen.

9. Vorrichtung gemäß Anspruch 1, wobei die mindestens eine geformte Nut (24, 25, 42, 60, 76) und der mindestens eine Vorsprung (26, 27, 78) eine Schwalbenschwanz-Konfiguration aufweisen.

10. Vorrichtung gemäß Anspruch 1, wobei das erste Element (1, 2, 37, 71, ...) eine sich durch es erstreckende längliche Öffnung (3, 4, 75) definiert, die eine Länge entlang der Längsachse (L) aufweist, wobei das Verbindungselement (22, 23, 39, 57, 77) einen sich durch es erstreckenden Durchgang (28, 29) definiert, wobei der zweite Endabschnitt (16, 17) des zweiten Elements (7, 8) sich durch die längliche Öffnung (3, 4, 75) und den Durchgang (28, 29) erstreckt und entlang der Länge der länglichen Öffnung (3, 4, 75) in einer unbegrenzten Anzahl von Positionen positionierbar ist, wobei sich ein Abschnitt des zweiten Endabschnitts (16, 17) von dem Verbindungsstück (22, 23, 39, 57, 77) erstreckt, wobei das Verriegelungselement (21, 59) mit dem Abschnitt des zweiten Endabschnitts (16, 17) zum Verriegeln des zweiten Endabschnitts (16, 17) in einer bestimmten Position entlang der Länge der länglichen Öffnung (3, 4, 75) in Eingriff ist.

11. Vorrichtung gemäß Anspruch 10, wobei das erste Element (1, 2, 37, 71, ...) ein Paar von Gleitflächen (24, 25, 42, 60, 76) definiert, die sich entlang der Längsachse erstrecken, wobei jede der Gleitflächen (24, 25, 42, 60, 76) auf gegenüberliegenden Seiten der Öffnung (3, 4, 75) angeordnet ist, wobei das Verbindungselement (22, 23, 39, 57, 77) ein Paar von seitlichen Rändern (26, 27, 78) definiert, wobei jeder der seitlichen Ränder (26, 27, 78) ein Profil aufweist, das im Wesentlichen komplementär zu einer entsprechenden der Gleitflächen (24, 25, 42, 60, 76) ist, wobei das Paar von seitlichen Rändern (26, 27, 78) mit dem Paar von Gleitflächen (24, 25, 42, 60, 76) in Eingriff ist, um zu erlauben, dass das Verbindungselement (22, 23, 39, 57, 77) relativ zu dem ersten Element (1, 2, 37, 71, ...) in einer Richtung entlang der Längsachse (L) gleitet.

12. Vorrichtung gemäß Anspruch 11, wobei das Paar von seitlichen Rändern (26, 27, 78) mit dem Paar von Gleitflächen (24, 25, 42, 60, 76) in einer Schwalbenschwanz-Konfiguration in Eingriff ist, wobei die Schwalbenschwanz-Konfiguration es ermöglicht, dass das Verbindungselement (22, 23, 39, 57, 77) relativ zu dem ersten Element (1, 2, 37, 71, ...) in Richtung entlang der Längsachse (L) gleitet, während das Verbindungselement (22, 23, 39, 57, 77) auf einer sich längs erstreckenden Fläche des ersten Elements (1, 2, 37, 71, ...) gefangen ist.

13. Vorrichtung gemäß Anspruch 10, wobei der Durchgang (28, 29) des Verbindungselements (22, 23, 39, 57, 77) gestaltet ist zum Aufnehmen des zweiten Endabschnitts (16, 17) des zweiten Elements (7, 8) in einem variablen Winkel relativ zu dem ersten Element (1, 2, 37, 71, ...), wobei der Durchgang (28, 29) sich auf eine im Wesentlichen kugelförmig geformte Führungsfläche öffnet, wobei das Verriegelungselement (21, 59) eine zweite im Wesentlichen kugelförmig geformte Führungsfläche definiert, die im Wesentlichen komplementär zu der ersten im Wesentlichen kugelförmig geformten Führungsfläche ist, wobei das Verriegelungselement (21, 59) mit dem Abschnitt des zweiten Endabschnitts (16, 17) in Eingriff ist und die erste und die zweite im Wesentlichen kugelförmige Fläche zusammendrückt, um den zweiten Endabschnitt (16, 17) in einem bestimmten Winkel relativ zu dem ersten Element (1, 2, 37, 71, ...) zu verriegeln.

14. Vorrichtung gemäß Anspruch 10, wobei der Abschnitt des zweiten Endabschnitts (16, 17) ein Gewinde aufweist und das Verriegelungselement (21, 59) eine Sicherungsmutter ist.

15. Vorrichtung gemäß Anspruch 14, wobei die Sicherungsmutter (59) einen Abbruch-Abschnitt (61b) aufweist, der von dem verbleibenden Abschnitt (61a) der Sicherungsmutter (59) bei Aufbringen einer vorbestimmten Menge von Drehkraft auf den Abbruch-Abschnitt (61b) getrennt wird.

16. Vorrichtung gemäß Anspruch 10, wobei das zweite Element (7, 8) eine Schulter (12, 13, 54) aufweist, die zwischen dem ersten (9, 11) und dem zweiten (16, 17) Endabschnitt angeordnet ist, wobei die Schulter (12, 13) einer ersten Seite des ersten Elements (1, 2, 37, 71, ...) zugewandt ist, wenn der zweite Endabschnitt (16, 17) durch die längliche Öffnung (3, 4, 75) hindurchgeführt wird, wobei das Verbindungselement (22, 23, 39, 57, 77) auf einer entgegengesetzten zweiten Seite des ersten Elements (1, 2, 37, 71, ...) angeordnet ist, wobei die Schulter (12, 13, 54) gestaltet ist zum Drücken gegen die erste Seite des ersten Elements (1, 2, 37, 71, ...) zum Verriegeln des zweiten Elements (7, 8) in der bestimmten Position entlang der Länge der länglichen Öffnung (3, 4, 75).

17. Vorrichtung gemäß Anspruch 10, ferner ein anderes von den Verbindungselementen (22, 23, 39, 57) aufweisend, das formschlüssig auf einer entgegengesetzten Seite des ersten Elements (1, 2, 37, ...) in einer Richtung (P) im Wesentlichen senkrecht zu der Längsachse (L) gehalten wird, wobei der zweite Endabschnitt (16, 17) des zweiten Elements (7, 8) durch die längliche Öffnung (3, 4) und jeden der Durchgänge (28, 29) in dem Paar von Verbindungselementen (22, 23, 39, 57) hindurchgeht und variabel entlang der Länge der länglichen Öffnung (3, 4) positionierbar ist, wobei das Verriegelungselement (21, 59) mit dem Abschnitt des zweiten Endabschnitts (16, 17) zum Verriegeln des zweiten Endabschnitts (16, 17) in der bestimmten Position entlang der Länge der länglichen Öffnung (3, 4) in Eingriff ist.

18. Vorrichtung gemäß Anspruch 1, wobei das erste Element eine längliche Platte (1, 2, 37, 56, 62, 63) ist, deren Länge sich entlang der Längsachse (L) erstreckt und die entlang der Wirbelsäule positionierbar ist.

19. Vorrichtung gemäß Anspruch 1, ferner ein längliches Stabelement (70) aufweisend, das entlang der Wirbelsäule positionierbar ist, wobei das erste Element (71) eine Öffnung (73) definiert, die sich durch es in einer Richtung quer zu der Längsachse (L) erstreckt und eine Größe zum Aufnehmen des länglichen Elements (70) zum Querverbinden des Stabelements (70) mit dem zweiten Element (7) aufweist.

20. Vorrichtung gemäß Anspruch 1, ferner eine Mehrzahl der ersten Elemente (1, 2, 37) aufweisend, wobei die Mehrzahl von ersten Elementen (1, 2, 37) einstückig gebildet ist, um ein Einzelstück zu definieren, das sich entlang der Längsachse (L) erstreckt, wobei das Einzelstück einen abgeschwächten Bereich (6) zwischen benachbarten der Mehrzahl der ersten Elemente (1, 2, 37) definiert, zum Erleichtern des Biegens des Einzelstücks entlang der Längsachse, wobei jedem der Mehrzahl von ersten Elementen (1, 2, 37) jeweilige des zweiten Elements (7, 8), des Verbindungselements (22, 23, 39, 57) und des Verriegelungselements (21) zugeordnet sind.

21. Vorrichtung gemäß Anspruch 20, wobei der geschwächte Bereich eine Kerbe (6) aufweist, die sich quer über das Einzelstück in einer Richtung im Wesentlichen senkrecht zu der Längsachse (L) erstreckt.

22. Vorrichtung gemäß Anspruch 21, wobei die Kerbe (6) auf der gleichen Seite des Einzelstücks angeordnet ist, wie das Verriegelungselement (21).

23. Vorrichtung gemäß Anspruch 1, wobei das erste Element (1, 2) eine obere und eine untere Fläche und ein Paar von seitlich zugewandten Seiten (1a, 2a) definiert, die sich dazwischen erstrecken, wobei das Verbindungselement (22, 23) benachbart zu der oberen Fläche angeordnet ist und ein Paar von seitlich zugewandten abgeflachten Abschnitten (22a, 23a) aufweist, die im Wesentlichen in der gleichen Ebene liegen wie das Paar von seitlich zugewandten Seiten (1a, 2a).

24. Vorrichtung gemäß Anspruch 1, wobei das erste Element (1, 2, 37, 71, ...) entlang der Wirbelsäule positionierbar ist, wobei die Längsachse (L) im Wesentlichen parallel zu einer Sagittalebene ausgerichtet ist.

25. Vorrichtung gemäß Anspruch 1, ferner mindestens zwei der ersten Elemente (1, 2, 62, 63) aufweisend, die Ende zu Ende entlang der Längsachse (L) angeordnet sind, wobei eines (1, 62) der ersten Elemente einen Kugel-Abschnitt (35) aufweist und ein anderes benachbartes (2, 63) der ersten Elemente einen Fassungsabschnitt (36) aufweist, der zum Aufnehmen des Kugel-Abschnitts (35) gestaltet ist, wobei der Kugel-Abschnitt (35) zum Bereitstellen relativer Gelenkigkeit zwischen den mindestens zwei ersten Elementen (1, 2, 62, 63) in drei Dimensionen in den Fassungsabschnitt (36) eingesetzt ist.

26. Vorrichtung gemäß Anspruch 25, wobei jedes der mindestens zwei ersten Elemente (63) einen der Kugel-Abschnitte (35), der benachbart zu einem Ende davon angeordnet ist, sowie einen der Fassungsabschnitte (36), der benachbart zu einem entgegengesetzten zweiten Ende davon angeordnet ist, aufweist.

27. Vorrichtung gemäß Anspruch 25, ferner Mittel zum Halten des Kugel-Abschnitts (35) in dem Fassungsabschnitt (36) und zum Beibehalten der mindestens zwei der ersten Elemente (1, 2, 62, 63) in einer bestimmten Ausrichtung relativ zueinander aufweisend.

28. Vorrichtung gemäß Anspruch 27, wobei das Haltemittel einen Ring aufweist, der eine Größe zum Aufnehmen des Fassungsabschnitts (36) hat und der zumindest teilweise aus einem Form-Erinnerungsmaterial geformt ist.

29. Vorrichtung gemäß Anspruch 1, wobei das Verbindungselement (22, 23, 39, 77) in einer unbegrenzten Anzahl von Positionen entlang der Längsachse (L) des ersten Elements (1, 2, 37, 71, ...) positionierbar ist.

30. Vorrichtung gemäß Anspruch 1, wobei das erste Element (1, 2, 37, 71, ...) eine erste Seite und eine zweite entgegengesetzte Seite aufweist, die sich jeweils entlang der Längsachse (L) erstrecken, wobei das erste Element (1, 2, 37, 71) eine Öffnung (3, 4, 75) definiert, die sich zwischen der ersten und der zweiten Seite erstreckt, wobei das Verbindungselement (22, 23, 39, 57, 77) einen Durchgang (28, 29) definiert, der sich durch es erstreckt und eine Größe zum Aufnehmen des zweiten Endabschnitts (16, 17) des zweiten Elements (7, 8) hat, wobei das Verbindungselement (22, 23, 39, 57, 77) benachbart zu der ersten Seite angeordnet ist, wobei die erste Seite mit dem Durchgang (28, 29) zu der Öffnung (3, 4, 75) ausgerichtet werden kann, und
wobei das zweite Element (7, 8) eine Schulter (12, 13, 54) aufweist, die zwischen dem ersten (9, 11) und dem zweiten (16, 17) Endabschnitt angeordnet ist, wobei die Schulter (12, 13, 54) zur Abstützung an der zweiten Seite des ersten Elements (1, 2, 37, 71, ...), wenn der zweite Endabschnitt (16, 17) durch die Öffnung (3, 4, 75) und den Durchgang (28, 29) hindurchgeführt wird, gestaltet ist zum Unterstützen bei dem Halten des zweiten Endabschnitts (16, 17) in der bestimmten Position entlang der Längsachse (L).

31. Vorrichtung gemäß Anspruch 30, wobei die Schulter (54) mindestens eine zylinderförmige Führungsfläche (52) definiert, die sich seitlich von dem zweiten Element (7, 8) erstreckt, wobei die zweite Seite des ersten Elements (56, 62) eine Mehrzahl von zylinderförmigen Führungsflächen (55) definiert, die im Wesentlichen komplementär zu der mindestens einen zylinderförmigen Führungsfläche (52) sind, wobei die Mehrzahl von zylinderförmigen Führungsflächen (52) entlang der Längsachse (L) benachbart zu der Öffnung (3, 4) angeordnet ist und sich in einer Richtung (P) im Wesentlichen senkrecht zu der Längsachse (L) erstreckt, und
wobei die mindestens eine zylinderförmige Führungsfläche (52) zur Abstützung an einer entsprechenden der Mehrzahl von zylinderförmigen Führungsflächen (55) zum Unterstützen beim Halten des zweiten Endabschnitts (16, 17) in der bestimmten Position entlang der Längsachse (L) und zum Begrenzen der Winkelstellung des zweiten Elements (7, 8) relativ zu dem ersten Element (56, 62) in einer Ebene, die im Wesentlichen parallel zu der Längsachse (L) ausgerichtet ist, gestaltet ist.

32. Vorrichtung gemäß Anspruch 31, wobei die Schulter (54) ein Paar von der mindestens einen zylinderförmigen Führungsfläche (52) definiert, wobei sich jedes von dem zweiten Element (7, 8) in entgegengesetzten Richtungen erstreckt, wobei das erste Element (56, 62) entsprechende Paare der Mehrzahl von zylinderförmigen Führungsflächen (55) definiert, die jeweils entlang gegenüberliegenden Seiten der Öffnung (3, 4) angeordnet sind, und
wobei das Paar der mindestens einen zylinderförmigen Führungsfläche (52) gestaltet ist zur Abstützung an einer jeweiligen der entsprechenden Paare der Mehrzahl von zylinderförmigen Führungsflächen (55) zum Unterstützen beim Halten des zweiten Endabschnitts (16, 17) in der bestimmten Position entlang der Längsachse (L) und zum Unterstützen bei dem Begrenzen der Winkelstellung des zweiten Elements (7, 8) relativ zu dem ersten Element (56, 62) in einer Ebene, die im Wesentlichen parallel zu der Längsachse (L) ist.

33. Vorrichtung gemäß Anspruch 31, wobei die mindestens eine zylinderförmige Führungsfläche (52) konvex ist und wobei die Mehrzahl von zylinderförmigen Führungsflächen (55) konkav ist.

34. Vorrichtung gemäß Anspruch 30, wobei die Schulter (12, 13) eine kugelförmige Führungsfläche (14, 15) definiert, wobei das erste Element (1, 2) eine Mehrzahl von Aussparungen (80, 33) definiert, die im Wesentlichen komplementär zu der kugelförmigen Führungsfläche (14, 15) sind, wobei die Aussparungen (80, 33) entlang der Öffnung (3, 4) benachbart zu der zweiten Seite des ersten Elements (1, 2) angeordnet sind, und
wobei die kugelförmige Führungsfläche (14, 15) gestaltet ist zur Abstützung an einer entsprechenden der Mehrzahl von Aussparungen (80, 33) zum Unterstützen beim Halten des zweiten Endabschnitts (16, 17) in der bestimmten Position entlang der Längsachse (L).

35. Vorrichtung gemäß Anspruch 1, ferner ein anderes der Verbindungselemente (22, 23, 39) aufweisend, wobei das erste Element (1, 2, 37) entgegengesetzte Seiten aufweist und eine Öffnung (3, 4) definiert, die sich dazwischen erstreckt, wobei die Verbindungselemente (22, 23, 39) auf den entgegengesetzten Seiten des ersten Elements (1, 2, 37) angeordnet sind, wobei ein erstes (22) der Verbindungselemente (22, 23) einen ersten Durchgang (28) definiert, der sich durch es erstreckt und eine Größe hat zum Aufnehmen des zweiten Endabschnitts (16) des zweiten Elements in einem variablen Winkel relativ zu dem ersten Element (1, 2, 37), wobei sich der Durchgang (28) an einer ersten im Wesentlichen kugelförmigen Führungsfläche öffnet, wobei ein zweites (23) der Verbindungselemente (22, 23) einen zweiten Durchgang (29) definiert, der sich durch es erstreckt und eine Größe hat zum Aufnehmen des zweiten Endabschnitts (17) des zweiten Elements (8) in einem variablen Winkel relativ zu dem ersten Element (1, 2, 37), wobei sich der zweite Durchgang (29) an einer zweiten im Wesentlichen kugelförmigen Führungsfläche öffnet,
wobei das Verriegelungselement (21) eine dritte im Wesentlichen kugelförmige Führungsfläche definiert, die im Wesentlichen komplementär zu der ersten kugelförmigen Führungsfläche (31) des ersten Verbindungselements (22) ist,
wobei das zweite Element (7) eine Schulter aufweist, die zwischen dem ersten (9) und dem zweiten (16) Endabschnitt angeordnet ist, wobei die Schulter eine vierte im Wesentlichen kugelförmige Führungsfläche (14) definiert, die im Wesentlichen komplementär zu der zweiten im Wesentlichen kugelförmigen Führungsfläche des ersten Verbindungselements ist, und
wobei sich der zweite Endabschnitt (16) des zweiten Elements (7) durch die Öffnung (3) und den ersten (28) und den zweiten (29) Durchgang in dem ersten (22) und dem zweiten (23) Verbindungselement erstreckt, wobei das Verriegelungselement (21) mit dem Verbindungselement (22, 23) und dem zweiten Endabschnitt (16) zusammenwirkt für einen engen Eingriff der ersten (31) und der dritten im Wesentlichen kugelförmigen Führungsfläche mit der zweiten und der vierten im Wesentlichen kugelförmigen Führungsfläche zum Verriegeln des zweiten Endabschnitts (16) an der bestimmten Position entlang der Längsachse (L) und in einem bestimmten Winkel relativ zu der Längsachse (L).

36. Vorrichtung gemäß Anspruch 35, wobei das erste (22) und das zweite (23) Verbindungselement formschlüssig auf dem ersten Element (1, 2, 37) in einer Richtung (P) im Wesentlichen senkrecht zu der Längsachse (L) gehalten werden.

37. Vorrichtung gemäß Anspruch 1, wobei
das erste Element (1, 2, 37) eine Platte ist,
das zweite Element (7, 8) eine Knochenverankerung ist,
das Verbindungselement (22, 23, 39, 57, 77) zum Positionieren des Verbindungselements (22, 23, 39, 57, 77) in einer unbegrenzten Anzahl von Positionen entlang der Längsachse (L) entlang einer sich längs erstreckenden Fläche der Platte (1, 2, 37, ...) gleiten kann.

38. Vorrichtung gemäß Anspruch 37, wobei das Verbindungsstück (22, 23, 39, 57, 77) ein Paar von entgegengesetzten sich seitlich erstreckenden Vorsprüngen (26, 27, 78) definiert, die auf entgegengesetzten Seiten der sich längs erstreckenden Fläche der Platte (1, 2, 37, ...) zum Halten des Verbindungselements (22, 23, 39, 57, 77) in gleitendem Eingriff mit der Platte (1, 2, 37, ...) angeordnet sind.

39. Vorrichtung gemäß Anspruch 38, wobei die sich seitlich erstreckenden Vorsprünge (26, 27) des Verbindungselements (22, 23) mit im Wesentlichen komplementären sich seitlich erstreckenden Nuten (24, 25) zusammenwirken, die zum Bereitstellen einer schwalbenschwanzartigen Montier-Anordnung zwischen dem Verbindungselement (12, 23) und der Platte (1, 2) in der Platte (1, 2) definiert sind.

40. Vorrichtung gemäß Anspruch 37, wobei das Verbindungselement (22, 23, 39, 57, 77) und die Platte (1, 2, 37, ...) zusammenwirkende Flächenmittel zum Positionieren des Verbindungselements (22, 23, 39, 57, 77) in der unbegrenzten Anzahl von Positionen entlang der Längsachse (L) aufweisen, während das Verbindungselement (22, 23, 39, 57, 77) formschlüssig auf der Fläche der Platte (1, 2, 37, ...) gehalten wird.

41. Vorrichtung gemäß Anspruch 37, wobei der Knochenanker (7, 8) und die Platte (1, 2, 37, ...) Mittel zum Schwenken des Knochenankers (7, 8) durch einen Bereich von Winkeln relativ zu der Längsachse (L) der Platte (1, 2, 37, ...) aufweisen.

42. Vorrichtung gemäß Anspruch 37, ferner Mittel zum gelenkigen Verbinden einer Mehrzahl der Platten (62, 63) Ende zu Ende entlang der Längsachse (L) aufweisend.

43. Vorrichtung gemäß Anspruch 1, wobei
das erste Element (1, 2, 37, 71, ...) eine längliche Öffnung (3, 4, 75) definiert mit einer Länge in einer Längsrichtung (L),
das zweite Element (7, 8) eine Schraube mit einem Knochenverankerungsschaft (9, 11) und einem entgegengesetzten Endabschnitt (16, 17) ist, wobei der Knochenverankerungsschaft (9, 11) eingerichtet ist zum Eingreifen in einen Wirbelkörper, wobei der entgegengesetzte Endabschnitt (16, 17) eine Größe hat zum Durchgehen durch die längliche Öffnung (3, 4, 75), und
wobei das Verriegelungselement (21) die Schraube (7, 8) in Bezug auf das erste Element (1, 2, 37, 71, ...) in der bestimmten Position entlang der Längsachse (L) und mit einer bestimmten Winkelausrichtung relativ zu der Längsachse (L) verriegelt, wobei das Verriegelungselement (21) mindestens ein Gleitstück (22, 23) aufweist, das gestaltet ist zum Gleiten auf entsprechenden Gleitflächen (24, 25), die auf dem ersten Element (1, 2, 37, 71, ...) in der Längsrichtung (L) vorgesehen sind, wobei das mindestens eine Gleitstück (22, 23) eine Bohrung (28, 29) definiert, die eine Größe hat zum Aufnehmen des Endabschnitts (16, 17) der Schraube (7, 8), wobei der Endabschnitt (16, 17) der Schraube (7, 8) gestaltet ist zum Zusammenwirken mit dem Verriegelungselement (21) zum Verriegeln der Schraube (7, 8) in der bestimmten Längsposition und mit der bestimmten Winkelausrichtung.

## Revendications

1. Dispositif d'ostéosynthèse rachidienne, comprenant :
un premier élément (1, 2, 7, 71...) ayant un axe longitudinal (L) et étant positionnable de manière adjacente à la colonne vertébrale ;
un deuxième élément (7, 8) ayant une première partie d'extrémité (9, 11) configurée pour mettre en prise un corps vertébral et une deuxième partie d'extrémité (16, 17) ;
un élément de raccord (22, 23, 39, 57, 77) raccordant ladite deuxième partie d'extrémité (16, 17) dudit deuxième élément (7, 8) audit premier élément (1, 2, 37, 71...) dans une position variable le long dudit axe longitudinal (L), ledit élément de raccord (22, 23, 39, 57, 77) étant retenu de manière positive par rapport audit premier élément (1, 2, 37, 71...) dans une direction (P) sensiblement perpendiculaire audit axe longitudinal (L), l'un parmi ledit premier élément (1, 2, 37, 71...) et ledit élément de raccord (22, 23, 39, 57, 77) comprenant au moins une rainure profilée (24, 25, 42, 60, 76) s'étendant le long dudit axe longitudinal (L) et un autre parmi ledit premier élément (1, 2, 37, 71...) et ledit élément de raccord (22, 23, 39, 57, 77) comprenant au moins une saillie profilée (26, 27, 78) configurée sensiblement de manière complémentaire à ladite rainure profilée (24, 25, 42, 60, 76), ladite saillie profilée, (26, 27, 78) étant mise en prise de manière coulissante dans ladite rainure profilée (24, 25, 42, 60, 76) **caractérisé en ce que** ladite saillie profilée et ladite rainure profilée permettent audit élément de raccord (22, 23, 39, 57, 77) de coulisser par rapport audit premier élément (1, 2, 37, 71...) dans une direction le long dudit axe longitudinal (L), et coopèrent pour permettre audit élément de raccord (22, 23, 39, 57, 77) de coulisser le long d'une face longitudinale dudit premier élément (1, 2, 37, 71...) tout en étant capturée sur ledit premier élément (1, 2, 37, 71...) dans une direction (P) sensiblement perpendiculaire audit axe longitudinal (L), et **en ce qu'**un élément de verrouillage (21, 59) coopère avec ledit élément de raccord (22, 23, 39, 57, 77) et ladite deuxième partie d'extrémité (16, 17) pour verrouiller ladite deuxième partie d'extrémité (16, 17) dans une position particulière le long dudit axe longitudinal (L).

2. Dispositif selon la revendication 1, **caractérisé en ce qu'**il possède une paire de rainures profilées (24, 25, 42, 60, 76) et une paire de saillies profilées (26, 27, 78).

3. Dispositif selon la revendication 1, dans lequel lesdits éléments de raccord (22, 23, 39, 57, 77) possèdent un passage (28, 29) s'étendant à travers eux et étant configuré pour y recevoir ladite deuxième partie d'extrémité (16, 17) dudit deuxième élément (7, 8) selon un angle variable par rapport audit premier élément (1, 2, 37, 71...), ledit passage (28, 29) s'ouvrant sur une première surface d'appui de forme sensiblement sphérique, ledit élément de verrouillage (21, 59) définissant une deuxième surface d'appui de forme sensiblement sphérique sensiblement complémentaire à ladite première surface d'appui de forme sphérique, ledit élément de verrouillage (21, 59) coopérant avec ledit élément de raccord (22, 23, 39, 57, 77) et ladite deuxième partie d'extrémité (16, 17) pour mettre en prise étroitement lesdites première et deuxième surfaces d'appui sphériques et verrouiller ladite deuxième partie d'extrémité (16, 17) selon un angle particulier par rapport audit premier élément (1, 2, 37, 71...).

4. Dispositif selon la revendication 3, dans lequel ledit passage (28, 29) est de forme sphérique.

5. Dispositif selon la revendication 3, dans lequel ledit passage (28, 29) est de forme conique.

6. Dispositif selon la revendication 1, dans lequel ledit premier élément définit une paire de butées disposées le long dudit axe longitudinal (L) sur des côté opposés dudit élément de raccord (22, 23, 39, 57) pour maintenir ledit élément de raccord (22, 23, 39, 57) en engagement coulissant avec ledit premier élément (1, 2, 37).

7. Dispositif selon la revendication 6, dans lequel lesdites butées (10) sont des protubérances embouties.

8. Dispositif selon la revendication 1, dans lequel ladite rainure profilée (24, 24, 42, 60) et ladite saillie profilée (26, 27, 28) définissent des aspérités pour résister au coulissement dudit élément de raccord (22, 23, 39, 57), par rapport audit premier élément (1, 2, 37, 71...).

9. Dispositif selon la revendication 1, dans lequel ladite au moins une rainure profilée (24, 25, 42, 60, 76) et ladite au moins une saillie (26, 27, 78) possèdent une configuration en queue d'aronde.

10. Dispositif selon la revendication 1, dans lequel ledit premier élément (1, 2, 37, 71...) définit une ouverture oblongue (3, 4, 75) s'étendant à travers lui et ayant une longueur s'étendant le long dudit axe longitudinal (L), ledit élément de raccord (22, 23, 39, 57, 77) définissant un passage (28, 29) s'étendant à travers, ladite deuxième partie d'extrémité (16, 17) dudit deuxième élément (7, 8) passant à travers ladite ouverture oblongue (3, 4, 75) et ledit passage (28, 29) et étant positionnable le long de ladite longueur de ladite ouverture oblongue (3, 4, 75) dans un nombre infini de positions, une partie de ladite deuxième partie d'extrémité (16, 17) s'étendant à partir dudit connecteur (22, 23, 39, 57, 77), ledit élément de verrouillage (21, 59) mettant en prise ladite partie de ladite deuxième partie d'extrémité (16, 17) pour verrouiller ladite deuxième partie d'extrémité (16, 17) dans une position particulière le long de ladite longueur de ladite ouverture oblongue (3, 4, 75).

11. Dispositif selon la revendication 10, dans lequel ledit premier élément (1, 2, 37, 71...) définit une paire de glissières (24, 25, 42, 60, 76) s'étendant le long dudit axe longitudinal, chacune desdites glissières (24, 25, 42, 60, 76) étant disposée sur des côtés opposés de ladite ouverture (3, 4, 75) dudit élément de raccord (22, 23, 39, 57, 77) définissant une paire d'arêtes latérales (26, 27, 78), chacune desdites arêtes latérales (26, 27, 78) ayant un profil sensiblement complémentaire à l'une correspondante desdites glissières (24, 25, 42, 60, 76), ladite paire d'arêtes latérales (26, 27, 78) étant en prise avec ladite paire de glissières (24, 25, 42, 60, 76) pour permettre audit élément de raccord (22, 23, 39, 57, 77) de coulisser par rapport audit premier élément (1, 2, 37, 71...) dans une direction le long dudit axe longitudinal (L).

12. Dispositif selon la revendication 11, dans lequel ladite paire d'arêtes latérales (26, 27, 78) sont mises en prise avec ladite paire de glissières (24, 25, 42, 60, 76) dans une configuration en queue d'aronde, ladite configuration en queue d'aronde permettant audit élément de raccord (22, 23, 39, 57, 77) de coulisser par rapport audit premier élément (1, 2, 37, 71...) dans ladite direction le long dudit axe longitudinal (L) tout en capturant ledit élément de raccord (22, 23, 39, 57, 77) sur une face s'étendant longitudinalement dudit premier élément (1, 2, 37, 71...).

13. Dispositif selon la revendication 10, dans lequel ledit passage (28, 29) dudit élément de raccord (22, 23, 39, 57, 77) est configuré pour recevoir ladite deuxième partie d'extrémité (16, 17) dudit deuxième élément (7, 8) selon un angle variable par rapport audit premier élément (1, 2, 37, 71...), ledit passage (28, 29) s'ouvrant sur une première surface d'appui de forme sensiblement sphérique, ledit élément de verrouillage (21, 59) définissant une deuxième surface d'appui de forme sensiblement sphérique sensiblement complémentaire à ladite première surface d'appui de forme sensiblement sphérique, ledit élément de verrouillage (21, 59) mettant en prise ladite partie de ladite deuxième partie d'extrémité (16, 17) et comprimant étroitement lesdites première et deuxième surfaces d'appui sensiblement sphériques pour verrouiller ladite deuxième partie d'extrémité (16, 17) selon un angle particulier par rapport audit premier élément (1, 2, 37, 71...).

14. Dispositif selon la revendication 10, dans lequel ladite partie de ladite deuxième partie d'extrémité (16, 17) est filetée et ledit élément de verrouillage (21, 59) est un écrou de blocage.

15. Dispositif selon la revendication 14, dans lequel ledit écrou de blocage (59) comprend une partie de séparation (61b) qui est séparée de la partie restante (61a) dudit écrou de blocage (59) lors de l'application d'un couple d'intensité prédéterminée à ladite partie de séparation (61b).

16. Dispositif selon la revendication 10, dans lequel ledit deuxième élément (7, 8) possède un épaulement (12, 13, 54) disposé entre lesdites première partie d'extrémité (9, 11) et deuxième partie d'extrémité (16, 17), ledit épaulement (12, 13) faisant face à un premier côté dudit premier élément (1, 2, 37, 71...) quand ladite deuxième partie d'extrémité (16, 17) passe à travers ladite ouverture oblongue (3, 4, 75) ledit élément de raccord (22, 23, 39, 57, 77) étant disposé sur un deuxième côté opposé dudit premier élément (1, 2, 37, 71...) ledit épaulement (12, 13, 54) étant configuré pour prendre appui sur ledit premier côté dudit premier élément (1, 2, 37, 71...) pour verrouiller ledit deuxième élément (7, 8) dans ladite position particulière le long de ladite longueur de ladite ouverture oblongue (3, 4, 75).

17. Dispositif selon la revendication 10, comprenant en outre un autre desdits éléments de raccord (22, 23, 39, 57) maintenu positivement sur un côté opposé dudit premier élément (1, 2, 37, ...) dans une direction (P) sensiblement perpendiculaire audit axe longitudinal (L), ladite deuxième partie d'extrémité (16, 17) dudit deuxième élément (7, 8) passant à travers ladite ouverture oblongue (3, 4) et chacun desdits passages (28, 29) dans ladite paire d'éléments de raccord (22, 23, 39, 57) et étant positionnable de manière variable le long de ladite longueur de ladite ouverture oblongue (3, 4) dudit élément de verrouillage (21, 59) mettant en prise ladite partie de ladite deuxième partie d'extrémité (16, 17) pour verrouiller ladite deuxième partie d'extrémité (16, 17) dans ladite position particulière le long de ladite longueur de ladite ouverture oblongue (3, 4).

18. Dispositif selon la revendication 1, dans lequel ledit premier élément est une plaque allongée (1, 2, 37, 56, 62, 63) ayant une longueur s'étendant le long dudit axe longitudinal (L) et étant positionnable le long de la colonne vertébrale.

19. Dispositif selon la revendication 1, comprenant en outre un élément de tige allongée (70) positionnable le long de la colonne vertébrale, ledit premier élément (71) définissant une ouverture (73) s'étendant à travers dans une direction transversale audit axe longitudinal (L) et étant dimensionné pour recevoir ledit élément allongé (70) à l'intérieur pour raccorder transversalement ledit élément de tige (70) audit deuxième élément (7).

20. Dispositif selon la revendication 1, comprenant en outre une pluralité desdits premiers éléments (1, 2, 37), ladite pluralité de premiers éléments (1, 2, 37) étant formée d'un seul tenant pour définir une pièce unique s'étendant le long dudit axe longitudinal (L), ladite pièce unique définissant une région de moindre résistance (6) intermédiaire de ceux adjacents de ladite pluralité desdits premiers éléments (1, 2, 37) pour faciliter la courbure de ladite pièce unique le long dudit axe longitudinal, chacun de ladite pluralité de premiers éléments (1, 2, 37) étant associé avec ceux correspondants dudit deuxième élément (7, 8), dudit élément de raccord (22, 23, 39, 57) et dudit élément de verrouillage (21).

21. Dispositif selon la revendication 20, dans lequel ladite région de moindre résistance comprend une encoche (6) s'étendant à travers ladite pièce unique dans une direction sensiblement perpendiculaire audit axe longitudinal (L).

22. Dispositif selon la revendication 21, dans lequel ladite encoche (6) est disposée du même côté de ladite pièce unique que l'élément de verrouillage (21).

23. Dispositif selon la revendication 1, dans lequel ledit premier élément (1, 2) définit des surfaces supérieure et inférieure et une paire de côtés opposés latéralement (1a, 2a) s'étendant entre elles, ledit élément de raccord (22, 23) étant disposé de manière adjacente à ladite surface supérieure et comprenant une paire de parties aplaties opposées latéralement (22a, 23a) reposant sensiblement dans le même plan que ladite paire de côtés opposés latéralement (1a, 2a).

24. Dispositif selon la revendication 1, dans lequel ledit premier élément (1, 2, 37, 71...) est positionnable le long de la colonne vertébrale, ledit axe longitudinal (L) étant orienté sensiblement parallèlement à un plan sagittal.

25. Dispositif selon la revendication 1, comprenant en outre au moins deux desdits premiers éléments (1, 2, 62, 63) disposés bout à bout le long dudit axe longitudinal (L), l'un (1, 62) desdits premiers éléments comprenant une partie sphérique (35) et un autre élément adjacent (2, 63) desdits premiers éléments comprenant une partie de logement (36) configurée pour recevoir ladite partie sphérique (35) à l'intérieur, ladite partie sphérique (35) étant insérée dans ladite partie de logement (36) pour fournir une articulation relative entre lesdits au moins deux desdits premiers éléments (1, 2, 62, 63) en trois dimensions.

26. Dispositif selon la revendication 25, dans lequel chacun desdits au moins deux desdits premiers éléments (63) comprend une desdites parties sphériques (35) disposées de manière adjacente à une de ses extrémités et une desdites parties de logement (36) disposées de manière adjacente à une de ses deuxièmes extrémités opposées.

27. Dispositif selon la revendication 25, comprenant en outre des moyens pour retenir ladite partie sphérique (35) dans ladite partie de logement (36) et maintenir lesdits au moins deux desdits premiers éléments (1, 2, 62, 63) dans une orientation particulière les uns par rapport aux autres.

28. Dispositif selon la revendication 27, dans lequel ledit moyen de retenue comprend une bague dimensionnée pour recevoir ladite partie de logement (36) à l'intérieur et étant au moins partiellement formée d'un matériau à mémoire de forme.

29. Dispositif selon la revendication 1, dans lequel ledit élément de raccord (22, 23, 39, 77) est positionnable dans un nombre infini de positions le long dudit axe longitudinal (L) dudit premier élément (1, 2, 37, 71...).

30. Dispositif selon la revendication 1, dans lequel ledit premier élément (1, 2, 37, 71...) possède un premier côté et un deuxième côté opposé s'étendant chacun le long dudit axe longitudinal (L), ledit premier élément (1, 2, 37, 71) définissant une ouverture (3, 4, 75) s'étendant entre lesdits premier et deuxième côtés, ledit élément de raccord (22, 23, 39, 57, 77) définissant un passage (28, 29) s'étendant à travers et étant dimensionné pour recevoir ladite deuxième partie d'extrémité (16, 17) dudit deuxième élément (7, 8) à l'intérieur, ledit élément de raccord (22, 23, 39, 57, 77) étant disposé de manière adjacente audit premier côté avec ledit premier côté avec ledit passage (28,29) étant alignable avec ladite ouverture (3, 4, 75) ; et
où ledit deuxième élément (7, 8) possède un épaulement (12, 13, 54) disposé entre ladite première partie d'extrémité (9, 11) et ladite deuxième partie d'extrémité (16, 17), ledit épaulement (12, 13, 54) étant configuré pour prendre appui sur ledit deuxième côté dudit premier élément (1, 2, 37, 71...) quand ladite deuxième partie d'extrémité (16, 17) passe à travers ladite ouverture (3, 4, 75) et ledit passage (28, 29) pour aider à retenir ladite deuxième partie d'extrémité (16, 17) dans ladite position particulière le long dudit axe longitudinal (L).

31. Dispositif selon la revendication 30, dans lequel ledit épaulement (54) définit au moins une surface d'appui de forme cylindrique (52) faisant saillie latéralement depuis ledit deuxième élément (7, 8), ledit deuxième côté dudit premier élément (56, 62) définissant une pluralité de surfaces d'appui de forme cylindrique (55) sensiblement complémentaires à ladite au moins une surface d'appui de forme cylindrique (52), ladite pluralité de surfaces d'appui de forme cylindrique (52) étant disposées le long dudit axe longitudinal (L) adjacent à ladite ouverture (3, 4) et s'étendant dans une direction (P) sensiblement perpendiculaire audit axe longitudinal (L) ; et
où ladite au moins une surface d'appui de forme cylindrique (52) est configurée pour prendre appui sur l'une correspondante de ladite pluralité de surfaces d'appui de forme cylindrique (55) pour aider à retenir ladite deuxième partie d'extrémité (16, 17) à ladite position particulière, le long dudit axe longitudinal (L) et pour limiter l'angulation dudit deuxième élément (7, 8) par rapport audit premier élément (56, 62) dans un plan orienté sensiblement parallèlement audit axe longitudinal (L).

32. Dispositif selon la revendication 31, dans lequel ledit épaulement (54) définit une paire desdites au moins une surface d'appui de forme cylindrique (52) chacune s'étendant depuis ledit deuxième élément (7, 8) dans des directions opposées, ledit premier élément (56, 62) définissant des paires correspondantes de ladite pluralité de surfaces d'appui de forme cylindrique (55) chacune étant disposée le long de côtés opposés de ladite ouverture (3, 4) ; et
où ladite paire desdites au moins une surface d'appui de forme cylindrique (52) est configurée pour prendre appui sur l'une respective desdites paires correspondantes de ladite pluralité de surfaces d'appui de forme cylindrique (55) pour aider à retenir ladite deuxième partie d'extrémité (16, 17) à ladite position particulière le long dudit axe longitudinal (L) et pour limiter l'angulation dudit deuxième élément (7, 8) par rapport audit premier élément (56, 62) dans un plan orienté sensiblement parallèlement audit axe longitudinal (L).

33. Dispositif selon la revendication 31, dans lequel ladite au moins une surface d'appui de forme cylindrique (52) est convexe et ladite pluralité de surfaces d'appui de forme cylindrique (55) sont concaves.

34. Dispositif selon la revendication 30, dans lequel ledit épaulement (12, 13) définit une surface d'appui de forme sphérique (14, 15) ledit premier élément (1, 2) définissant une pluralité d'évidements (80, 33) sensiblement complémentaires à ladite surface d'appui de forme sphérique (14, 15), lesdits évidements (80, 33) étant disposés le long de ladite ouverture (3, 4) adjacente audit deuxième côté dudit premier élément (1, 2), et
où ladite surface d'appui de forme sphérique (14, 15) est configurée pour prendre appui sur un évidement correspondant de ladite pluralité d'évidements (80, 33) pour aider à retenir ladite deuxième partie d'extrémité (16, 17) à ladite position particulière le long dudit axe longitudinal (L).

35. Dispositif selon la revendication 1, comprenant en outre un autre desdits éléments de raccord (22, 23, 39), ledit premier élément (1, 2, 37) ayant des côtés opposés et définissant une ouverture (3, 4) s'étendant entre eux, lesdits éléments de raccord (22, 23, 39) étant disposés sur lesdits côtés opposés dudit premier élément (1, 2, 37), un premier élément (22) desdits éléments de raccord (22, 23) définissant un premier passage (28) s'étendant à travers et dimensionné pour recevoir ladite deuxième partie d'extrémité (16) dudit deuxième élément (7) à l'intérieur selon un angle variable par rapport audit premier élément (1, 2, 37), ledit passage (28) s'ouvrant sur une première surface d'appui de forme sensiblement sphérique, un deuxième élément (23) desdits éléments de raccord (22, 23) définissant un deuxième passage (29) s'étendant à travers et dimensionné pour recevoir ladite deuxième partie d'extrémité (17) dudit deuxième élément (8) à l'intérieur selon un angle variable par rapport audit premier élément (1, 2, 37), ledit deuxième passage (29) s'ouvrant sur une deuxième surface d'appui de forme sensiblement sphérique ;
ledit élément de verrouillage (21) définissant une troisième surface d'appui de forme sensiblement sphérique sensiblement complémentaire à ladite première surface d'appui de forme sphérique (31) dudit premier élément de raccord (22) ;
ledit deuxième élément (7) ayant un épaulement disposé entre ladite première partie d'extrémité (9) et ladite deuxième partie d'extrémité (16), ledit épaulement définissant une quatrième surface d'appui de forme sensiblement sphérique (14) sensiblement complémentaire à ladite deuxième surface d'appui de forme sensiblement sphérique dudit deuxième élément de raccord ; et
ladite deuxième partie d'extrémité (16) dudit deuxième élément (7) s'étendant à travers ladite ouverture (3) et ledit premier passage (28) et ledit deuxième passage (29) dans ledit premier élément de raccord (22) et ledit deuxième élément de raccord (23), ledit élément de verrouillage (21) coopérant avec ledit élément de raccord (22, 23) et ladite deuxième partie d'extrémité (16) pour mettre en prise étroitement lesdites première (31) et troisième surfaces d'appui sensiblement sphériques et lesdites deuxième et quatrième surfaces d'appui sensiblement sphériques pour verrouiller ladite deuxième partie d'extrémité (16) à ladite position particulière le long dudit axe longitudinal (L) et selon un angle particulier par rapport audit axe longitudinal (L).

36. Dispositif selon la revendication 35, dans lequel ledit premier élément de raccord (22) et ledit deuxième élément de raccord (23) sont retenus de manière positive sur ledit premier élément (1, 2, 37) dans une direction (P) sensiblement perpendiculaire audit axe longitudinal (L).

37. Dispositif selon la revendication 1, dans lequel
ledit premier élément (1, 2, 37...) est une plaque,
ledit deuxième élément (7, 8) est une fixation osseuse,
ledit élément de raccord (22, 23, 39, 57, 77) est coulissable le long d'une face s'étendant longitudinalement de ladite plaque (1, 2, 37...) pour positionner ledit élément de raccord (22, 23, 39, 57, 77) dans un nombre infini de positions le long dudit axe longitudinal (L).

38. Dispositif selon la revendication 37, dans lequel ledit raccord (22, 23; 39, 57, 77) définit une paire de saillies opposées s'étendant latéralement (26; 27, 78) disposées sur des côtés opposés de ladite face s'étendant longitudinalement de ladite plaque (1, 2; 37...) pour maintenir ledit élément de raccord (22, 23, 39, 57, 77) en engagement coulissant avec ladite plaque (1, 2, 37...).

39. Dispositif selon la revendication 38, dans lequel lesdites saillies s'étendant latéralement (26, 27) dudit élément de raccord (22, 23) coopèrent avec des rainures s'étendant latéralement sensiblement complémentaires (24, 25) définies dans ladite plaque (1, 2) pour fournir un agencement de montage de type à queue d'aronde entre ledit élément de raccord (12, 23) et ladite plaque (1, 2).

40. Dispositif selon la revendication 37, dans lequel ledit élément de raccord (22, 23, 39, 57, 77) et ladite plaque (1, 2, 37...) comprennent des moyens de coopération de surface pour positionner ledit élément de raccord (22, 23, 39, 57, 77) dans ledit nombre infini de positions le long dudit axe longitudinal (L) tout en maintenant de manière positive ledit élément de raccord (22, 23, 39, 57, 77) sur ladite face de ladite plaque (1, 2, 37...).

41. Dispositif selon la revendication 37, dans lequel ladite fixation osseuse (7, 8) et ladite plaque (1, 2, 37...) comprennent des moyens pour faire pivoter ladite fixation osseuse (7, 8) selon une gamme d'angles par rapport audit axe longitudinal (L) de ladite plaque (1, 2, 37...).

42. Dispositif selon la revendication 37, comprenant en outre des moyens pour relier de manière articulée une pluralité desdites plaques (62, 63) bout à bout le long dudit axe longitudinal (L).

43. Dispositif selon la revendication 1, dans lequel
ledit premier élément (1, 2, 37, 71...) définit une ouverture oblongue (3, 4, 75) ayant une longueur s'étendant dans une direction longitudinale (L),
ledit deuxième élément (7, 8) est une vis ayant une tige d'ancrage osseux (9, 11) et une partie d'extrémité opposée (16, 17), ladite tige d'ancrage osseux (9, 11) étant configurée pour mettre en prise un corps vertébral, ladite partie d'extrémité opposée (16, 17) étant dimensionnée pour passer à travers ladite ouverture oblongue (3, 4, 75), et
ledit élément de verrouillage (21) verrouille ladite vis (7, 8) par rapport audit premier élément (1, 2, 37, 71...) Dans une dite position particulière le long dudit axe longitudinal (L) et avec une orientation angulaire particulière par rapport audit axe longitudinal (L) et ledit élément de verrouillage (21) comprenant au moins un patin (22, 23) configuré pour coulisser sur des glissières correspondantes (24, 25) prévues sur ledit premier élément (1, 2, 37, 71...) dans ladite direction longitudinale (L), ledit au moins un patin (22, 23) définissant un alésage (28, 29) dimensionné pour recevoir ladite partie d'extrémité (16, 17) de ladite vis (7, 8) à l'intérieur, ladite partie d'extrémité (16, 17) de ladite vis (7, 8) étant configurée pour coopérer avec ledit élément de verrouillage (21) pour verrouiller ladite vis (7, 8) dans ladite position longitudinale particulière et avec ladite orientation angulaire particulière.
